# EUROPEAN PATENT SPECIFICATION

(11) **EP 1 947 195 B1**
(45) Date of publication and mention of the grant of the patent: **10.11.2010**
(21) Application number: 06807950.8
(22) Date of filing: 29.08.2006
(51) Int. Cl.: C12Q 1/68, A61K 48/00, G01N 33/68

(54) **METHOD FOR THE IN VITRO DETECTION OF A PREDISPOSITION TO THE DEVELOPMENT OF ALTERATIONS IN OVARIAN FUNCTION**
VERFAHREN ZUM IN-VITRO-NACHWEIS EINER VERANLAGUNG ZUR ENTWICKLUNG VON VERÄNDERUNGEN DER OVARIENFUNKTION
METHODE DE DETECTION IN VITRO DE LA PREDISPOSITION A DEVELOPPER DES ALTERATIONS DE LA FONCTION OVARIENNE

(30) Priority: 13.09.2005 ES 200502225
(43) Date of publication of application: 23.07.2008
(73) Proprietor: Neocodex, S.L., 41092 Sevilla (ES)
(72) Inventor: MORÓN, Francisco J., E-41092 Sevilla (ES); ROYO, José L., E-41092 Sevilla (ES); SÁEZ, Maria Eugenia, E-41092 Sevilla (ES); REAL, Luis Miguel, E-41092 Sevilla (ES); RUIZ, Agustín, E-41092 Sevilla (ES)
(74) Representative: Illescas, Manuel
(86) International application number: PCT/ES2006/070130
(87) International publication number: WO 2007/031590

(56) References cited:
- WO-A-01/85926
- WO-A-03/102199
- WO-A1-00/50638
- DI PASQUALE E. ET AL.: 'Hypergonadotropic ovarian failure associated with an inherited mutation of human bone morphogenetic protein-15 (BMP15) gene' AM. J. HUM. GENET. vol. 75, no. 1, July 2004, pages 106 - 111, XP003010230
- MOORE R.K. ET AL.: 'Molecular biology and physiological role of the oocyte factor, BMP-15' MOL. CELL ENDOCRINOL. vol. 234, no. 1-2, 29 April 2005, pages 67 - 73, XP004844665
- DIXIT H. ET AL.: 'Missense mutations in the BMP15 gene are associated with ovarian failure' HUM. GENET. vol. 119, no. 4, May 2006, pages 408 - 415, XP019346334
- MCNATTY K.P. ET AL.: 'Oocyte-expressed genes affecting ovulation rate' MOL. CELL ENDOCRINOL. vol. 234, no. 1-2, 29 April 2005, pages 57 - 66, XP004844664

### SEQUENCE LISTING

<110> NEOCODEX
   <120> METHOD FOR THE IN VITRO DETECTION OF A PREDISPOSITION TO THE DEVELOPMENT OF ALTERATIONS IN OVARIAN FUNCTION
   <130> PCT-01112
   <160> 19
   <210> 1
   <211> 18
   <212> ADN
   <213> Artificial sequence
   <220>
   <221> Primer
   <223> PF
<400>
   agctcccata gattactc
<210> 2
   <211> 17
   <212> ADN
   <213> Artificial sequence
   <220>
   <221> Primer
   <223> PR1
<400>
   ctggaccaaa gttgcct
<210> 3
   <211> 20
   <212> ADN
   <213> Artificial sequence
   <220>
   <221> Primer
   <223> PF2
<400>
   ggtactatct tgttctcagc
<210> 4
   <211> 20
   <212> ADN
   <213> Artificial sequence
<220>
   <221> Primer
   <223> R2
<400>
   ttccttggct gttcgccagg
<210> 5
   <211> 20
   <212> ADN
   <213> Artificial sequence
   <220>
   <221> Primer
   <223> F2
<400>
   ccatccttgg ttgtggagcc
<210> 6
   <211> 21
   <212> ADN
   <213> Artificial sequence
   <220>
   <221> Primer
   <223> R5
<400>
   acaaggcaac aaagcctgac a
<210> 7
   <211 > 20
   <212> ADN
   <213> Artificial sequence
   <220>
   <221> Primer
   <223> e2F
<400>
   gaggtaagaa gctaaacctc
<210> 8
   <211> 20
   <212> ADN
   <213> Artificial sequence
   <220>
   <221> Primer
   <223> e2R
<400>
   tacaggatta cttgcagctt
<210> 9
   <211> 18
   <212> ADN
   <213> Artificial sequence
   <220>
   <221> Primer
   <223> PR3-B
   <220>
   <223> biotine marked
<400>
   agtgctccaa gaagttcc
<210> 10
   <211> 18
   <212> ADN
   <213> Artificial sequence
   <220>
   <221> Primer
   <223> S4
<400>
   caagattgag aaccactg
<210> 11
   <211> 20
   <212> ADN
   <213> Artificial sequence
   <220>
   <221> Primer
   <223> R2-B
   <220>
   <223> Biotine marked
<400>
   ttccttggct gttcgccagg
<210> 12
   <211> 17
   <212> ADN
   <213> Artificial sequence
   <220>
   <221> Primer
   <223> S2
<400>
   gcctgttgtt gaacact
<210> 13
   <211> 20
   <212> ADN
   <213> Artificial sequence
   <220>
   <221> Primer
   <223> F5
<400>
   cctggcgaac agccaaggaa
<210> 14
   <211> 21
   <212> ADN
   <213> Artificial sequence
   <220>
   <221> Primer
   <223> R5-B
   <220>
   <223> Biotine marked
<400>
   acaaggcaac aaagcctgac a
<210> 15
   <211> 15
   <212> ADN
   <213> Artificial sequence
   <220>
   <221> Primer
   <223> S3
<400>
   tgaagccctt gacca
<210> 16
   <211> 21
   <212> ADN
   <213> Artificial sequence
   <220>
   <221> Primer
   <223> F-B
   <220>
   <223> Biotine marked
<400>
   attgttgcag gacaacagtg g
<210> 17
   <211> 21
   <212> ADN
   <213> Artificial sequence
   <220>
   <221> Primer
   <223> R
<400>
   tcacagtaag ggttaggtac g
<210> 18
   <211> 18
   <212> ADN
   <213> Artificial sequence
   <220>
   <221> Primer
   <223> S
<400>
   catctcagac tagtgaag
<210> 19
   <211> 1085
   <212> ADN
   <213> Homo sapiens
   <220>
   <223> Promoter region closest to CAS and first 45 nucleotides of CDS for BMP15 gene
<220>
   <221> Recognition sequence of Xhol
   <222> 97 .. 102
   <220>
   <221> Recognition sequence of de Ncol
   <222> 1051 .. 1056
   <220>
   <221> Variant
   <222> 368
   <223> SNP-673C>T
   <220>
   <221> Variant
   <222> 1031
   <223> SNP -9C>G
   <220>
   <221> TATA Box
   <222> 192 .. 196
   <220>
   <221> Estrogen Response Element (ERE)
   <222> 977.. 992
<400>
   aacaaactag caaaataggc tgtccccagt gcaagtgcag gtgccagaac atttctctat        60
   cgataggtac cgagctctta cgcgtgctag cccgggctcg agagctccca tagattactc       120
   ttccccttcc tcaggcttct ctaatgaagc ccaataagga tctcaacacc ttccaaggta       180
   aaccagaaaa ttataagtca gagtccttaa acgtggctgc acattaaaat cacttgtgaa       240
   cttaaaaaaa tcctgatgca ctagttacaa cccaggccca ttacatcaga atggtaccca       300
   ggcattcata atagttaaag cttcccagat gattccaatc tacagccaag attgagaacc       360
   actgttacgg catcttcttc aagctccagc agccatactc agtagtttcc acattttagg       420
   ttttcatagg aacttcttgg agcactggta aagagccaga tttctggacg ccatcccaga       480
   gaatcctggt tcagacccag gagtctacat tcttaataat caccccagat agtttcactg       540
   cagattatta gcagacaata ttttgagaaa tgctgcatta gaggcatgga agtaggatgg       600
   gaacaggagg cctctggccc tttaaggatg gattaaggga ctacttttaa ggctgtaggg       660
   gcaagtagga tcaggctgct ttttttcatt ctagtggata agttaaggaa gtgggtcctg       720
   aaatggacct ctccttttaa gttacctgag cctctcaata ggtactatct tgttctcagc       780
   ccgggagagc gggaggggca tgctgccttg tcccaccttc tgtttctgtt tcttttgatg       840
   caaagaggcc aagttatgag gcaactttgg tccaggagat cctcttagag aaagcaacat       900
   agggcctgcc tgcctttctt tttcttgccc catccttggt tgtggagcca ggatgcagtt       960
   atctgcatga aaggaaatgg tcagagtgac gtcccttggg cttgtgttgg ggcctgttgt      1020
   tgaacactaa gcctttcaag atg gtc ctc ccc atg gaa gac gcc aaa aac ata      1073
   aag aaa ggc ccg

## Description

### Field of the invention

The present invention relates to employ of genetic markers to evaluate a predisposition to suffer from illnesses and disorders. Specifically, the present invention is based on the detection of SNPs within *BMP15* gene for the prediction to predispose alterations in the ovary function, and specifically in the ovulatory function, both natural (premature ovarian failure and early menopause) and iatrogenic (ovarian hyperstimulation syndrome, SHO) alterations.

### Background and technique state

The ovulation rate in mammals is determined by a complex exchange of hormonal signals between the pituitary gland and the ovary, and by a paracrine crosstalk between follicles, the oocyte and its adjacent somatic cells. To stimulate this function and to obtain better pregnancy rates, exogenous gonadotropins are widely used in assisted reproduction techniques (ART), during controlled ovarian stimulation (COS) protocols. Nowadays, recombinant follicle-stimulating hormone (rFSH) is the main treatment employed in ART (1). Several studies have shown a high variability in the clinical outcome of women undergoing rFSH treatment. This variability may depend on factors such as age of patients, ovarian reserve, or endocrine status (2). The use of the gonadotropin-releasing hormone agonists (GnRHa) is very important in ovarian stimulation protocols to suppress oestrogen production in the ovary via biochemical ablation of the Hypothalamus-Hypophysis-ovary axis (pituitary suppression). At present, rFSH dose calculation in a specific patient is performed empirically or on the basis of previous results in these or other patients. Besides, they don't apply any pharmacogenetic protocols neither to adjust the gonadotropins doses nor to prevent adverse effects associated to treatments. Nevertheless, this kind of studies is an emerging field in genetic research and it will drive to the development of predictive panels in response to COS (review in de Castro et al., (52)).

Usually, COS treatments give rise to different types of ovarian response, there being two extreme situations: patients with lower than normal response (poor responders) and patients with a high response to gonadotropins (high responders). Moreover, these last patients have a substantially higher risk of developing ovarian hyperstimulation syndrome and associated complications (3).

Ovarian hyperstimulation syndrome (OHSS) is a well-known iatrogenic complication (caused by medical treatment) of ovulation induction with gonadotropins and it can be accompanied by a severe morbidity that could be fatal. The clinical course of OHSS may involve, according to its severity and the occurrence of pregnancy, cardiovascular effects, electrolytic imbalance, pulmomary manifestations, neurohormonal and haemodynamic changes. It can also contribute to arteriolar vasodilatation, hepatic anomalies, hypoglobulinaemia, febrile morbidity, thromboembolism, neurological manifestations, and adnexal torsion (4). Therefore, the detection of patients with an increased risk of developing OHSS is a serious clinical problem, and multiple biomarkers in different and unrelated biochemical pathways have been evaluated as potential OHSS predictors. Between principal biomarkers we found oestradiol (5), renin angiotensin system (6, 7), cytokines (8-10), inhibins (11) and soluble selectins (12) mainly. Ovarian hyperstimulation entails very high serum oestradiol levels and altered progesterone/oestradiol ratios. So far, a limited number of fatal cases have been reported (13-16). This data is to stand out the importance of the prevention of OHSS and the potential utility of the predictive tools to anticipate the appearance.

Thereby, the study of genes involved in COS outcome with hormone treatments is really important and the pharmacogenetics approach is one of the useful tools to release it. There is enough evidence of genetic markers involved in poor response during COS treatment in women. Georgiu et al. studied common *Pvu*ll and rare *BstUI* restriction fragment length polymorphisms (RFLPs) within the *ESR1* gene and suggested that *ESR1* alleles might affect pregnancy rate and oocyte ratio during in vitro fertilization (IVF) (17). Later, Sundarrajan et al, partially, replicated this work and moreover, they found that *ESR1* Pvull PP carriers had lower follicle and oocyte numbers observed during COS (18).

Additionally, the Ser680Asn polymorphism in the *FSHR* gene was also associated with FSH basal level and elevated gonadotropin requirements during COS (19, 20). In preliminary studies, the applicant, NeoCodex, proposed that the same polymorphism at the *FSHR* Ser680Asn locus acts as a low-penetrance allele related to COS outcome. In this sense, this marker could be dependent on the basis of a complex multifactorial model (21). More recently, the applicant detected an oligogenic model, including specific *FSHR, ESR1* and *ESR2* genotype patterns, related to the poor response to rFSH hormone during COS treatment (22). Whereas, in relation with high response to COS, Daelmans et al, described that the same polymorphism (Ser680Asn) in the *FSHR* gene could be a predictor of severity of symptoms among OHSS patients (23). Furthermore, three different natural mutations in the *FSHR* gene were identified in patients presenting with recurrent severe spontaneous OHSS (not iatrogenic, that is, not caused by exogenous hormone during treatment) (24-26).

During our search for new candidate genes involved in COS outcome with hormone treatments, the *BMP15* gene has appeared as a good candidate gene, since, in animal models, different research groups have showed the significant role of this gene in ovarian development and folliculogenesis. A genetic study in sheep (one of the few species with mono-ovulation instead of multiple ovulation, like humans) provide evidence of the essential role of *BMP15* in folliculogenesis and fertility (27). Specifically, this study identified that Inverdale and Hanna sheep (which present the rare characteristic of a multiple ovulation with autosomal dominant models) have a natural mutation in the X chromosome (FecXⁱ and FecX^{H}). Females carrying homozygous mutations (hanna and inverdale) displayed follicular development arrests at the primary stage with an early block in folliculogenesis, resulting in infertility. In contrast, heterocygotes exhibited an increased ovulation rate and multiple pregnancies. Therefore, *bmp15* appears to be associated with mechanisms of infertility and super fertility in a dosage-sensitive manner (27).

Nowadays, five separate point mutations in the *bmp15* gene have been identified affecting the ovulation rate in ewes (27, 42, 43). Moreover, *bmp15* knock-out female mice are subfertile and show reduced ovulation rates after gonadotropin treatment (44). In this aspect, the analysis of the results obtained in both models suggests that *BMP15* may not affect follicular development and ovulation rate in the same way in all mammal species, and they request an interspecific comparative study. For this reason, it is not possible translate these discoveries in animal models to human being.

BMP15 protein is a member of the transforming growth factor-β (TGF-β) superfamily of proteins. This superfamily controls many aspects of development by binding and activating two types of transmembrane serine/threonine kinase receptors and SMAD proteins (intracellular proteins that mediate signals generated by other TGF-β related proteins via specific heteromeric complexes of transmembrane receptors) to regulate cellular functions (28). Given the relative importance of these factors in the intraovarian regulation of follicular development and its involvement in pregnancy rate, we considered *BMP15* to be a good candidate gene to be involved in alterations of the ovarian function in humans. This hypothesis is reinforced for a recent study that identified, in two sisters affected by hypergonadotropic ovarian failure (medical profile characterized by absence of menarche and menstrual period as well as puberty delay), a heterozygous nonconservative substitution (Y235C) in the pro-region of the *BMP15* allele (45). The Pro-region is an amino acid sequence located near the amino terminal of the recently translated precursor protein and it is removed during the postransductional processing, which give rise to a mature form (biologically active) of the protein. This mature form is important for a correct protein folding and, in the case of the TGF-β family, it seems to have influence on the formation of dimers. In keeping with this, the study establishes an association between the Y235C mutation and an impaired pro-protein processing of mature BMP15 form, the formation of abnormal dimerization protein (composed of precursor molecules and mature forms) and the lack of the stimulated activity in the granulosa cells proliferation. However, the study does not establish a relation between specific alleles within *BMP15* gene and the presence of a high response to FSH, OHSS, premature ovarian failure or early menopause. The identification of molecular markers to predict which patients will develop OHSS during COS cycles would permit a pharmacogenetic approximation to select the adjusted doses to each patient, helping thus, to prevent the appearance of OHSS.

Thereby, a clinical link has been found between an altered response to gonadotropins and the presence of early menopause and premature ovarian failure (52, 53), although the existence of a genetic basis for this phenomenon is uncertain. Consequently, the *BMP15* markers that affect COS outcome could give the chance of a therapeutic intervention in medical profiles related to women with age at menopause below normal range (about 50 years of age, being usually considered as high and low limits of normality interval values 45 and 55 years of age respectively). Moreover, they could be used to modify or to correct, ahead of time or as a preventive measure, the physiological parameters where *BMP15* gene is influencing. Thus, markers which would help to predict the predisposition to early menopause (women with age at menopause between 40 and 45 years of age) and premature ovarian failure (women with age at menopause bellows 40 years of age) and/or, a shorter reproductive period with lower than normal values (lower than 25 years), would give the chance to apply a correct therapy in patients with risk for these alterations mentioned, and to help to delay its appearance. The molecular markers of *BMP15* gene described in the invention complies both characteristics: they are valid in the prognosis of age at menopause and reproductive periods as well as in the prognosis of predisposition to develop OHSS during a controlled ovarian stimulation. The use of molecular markers within *BMP15* gene with that intention has an absolutely newness, having not found any antecedents in the literature of this approach.

### Description of the invention

The present invention applies the use of the molecular markers within *BMP15* gene in human, to predinically predict, the presence of OHSS and, high follicular production rates in women undergoing controlled ovarian stimulation. Furthermore, it could be possible to identify women with risk to present the age at menopause below the normal range and/or, to show a shorter reproductive periods regarding normal range in the population.

The basis of the invention is that *BMP15* determines the follicular recruitment induced by FSH. The presence of the specific *BMP15* alleles would suppose a predisposition to a higher follicular recruitment induced by FSH. So, on the one hand, women underwent FSH treatment carrying these alleles (as habitually occurs in assisted reproductive techniques inducing a controlled ovarian stimulation) would have a high predisposition to OHSS, even they would develop it ahead of time. On the other hand, women carrying these *BMP15* alleles could consume a high number of follicles per cycle in a natural way and thus, her ovarian reserve could get exhausted ahead of time. Since it is well known that the exhaustion of the follicular reserve is the cause of the appearance of menopause in women, the above-mentioned alleles could be very useful to predict an early exhaustion of the ovarian reserve.

In keeping with this, the described invention in the present patent supplies useful molecular markers of *BMP15* gene in OHSS prediction of patients underwent COS, providing a pharmacogenetic analysis in COS outcome. These same markers are also useful markers to predict the appearance of early menopause and premature ovarian failure, as well as to predict reproductive periods and age at menopause in women.

Just as it is used in the present invention, it is understood by molecular markers of *BMP15* gene the single nucleotide polymorphisms within this gene analysed in the present study in an independent manner, as well as, the combinations of these polymorphisms present in the same *BMP15* allele. These combinations are also mentioned in other points of the patent as "haplotype".

The invention also provides a method to predict the predisposition of alterations in the ovarian function by means of the detection of molecular markers within *BMP15* gene, specifically -673C>T, -9C>G, IVS1+905A>G and N103S markers, and the haplotypes of their combinations. From these markers, two (-673C>T and N103S) are not described as polymorphism in the data base up to now. On the other hand, -9C>G and IVS1+905A>G appear in the dbSNP list (data base of single nucleotide polymorphisms from NCBI, National Center for Biotechnology Information, in http://www.ncbi.nim.nih.gov/SNP/) and Hapmap of the Human Genome Project (http://www.hapmap.org) only. Although, for the time being, they had not been used for genetic studies and, they do not have any attached phenotype nor any described function. Its use as molecular markers associated to alternations in the ovarian function is totally innovative.

The utility of the invention is shown up with a genetic association study involved in COS outcome in 307 non-related women, with a normal ovarian function, who underwent COS using rFSH. In addition, we present a study with the same markers in 750 postmenopausal women proving the effect of the *BMP15* gene in age at menopause, its premature appearance (early menopause or premature ovarian failure) and the time of the reproductive periods. And with this, the utility of the detection of markers within *BMP15* gene for the prediction of alterations in the ovarian function related with mentioned phenomenon.

This study is compatible with previous researches in animals carrying a *BMP15* mutation, as in ewes. However, the molecular basis of molecular markers proposed in the present invention is different, since the FecX mutation described in ewes affects to *BMP15* coding region, while the TGG- haplotype, proposed as a marker, is a variant close to, but not localized within, the coding sequence of the human protein.

### Detailed description of the invention

### Pharmacogenetic study of COS

### Patients

The study population was 307 Caucasian women undergoing In-Vitro Fertilization (IVF) or Intracytoplasmic Sperm Injection (ICSI) techniques. The mean ± SD age of patients was 32.6 ± 2.56 years. The causes of infertility in selected women were 174 male factors, 90 tubal factors or 43 both of them. Ovulation in all patients included was conserved. Evaluation of the response to FSH in women was performed using standard criteria and parameters previously published by Neocodex (21).

In this study, we included women underwent COS due to male factors, because they are a good reference like controls, since these women have a conserved ovarian function. The key of this study is the variability in the ovarian outcome to treatment, for that reason, it is better to use women with normal ovaries than women with pathological ovaries. The healthy women underwent fertilisation treatments, being men the affected ones due to fertilisation problems (50% of cases approximately), are a perfect group to analyze variations in the response to ovarian stimulation treatments.

The study design is retrospective. All patients were treated with rFSH to achieve ovarian stimulation. Gonadotropin-releasing hormone (GnRH) analogue was administrated to all women included in our study, according to the minimal monitoring method described by Wikland et al. (29). Specifically, all patients were down regulated (pituitary suppression) with a GnRH agonist, according to a long protocol, permitting fairly precise programming of the oocyte collection (triptorelin 0.1 mg/day over 15 days to reach oestradiol values ≤40pmol/L). Once minimal oestradiol values were obtained, the women were treated with 100-200 IU/day of human rFSH for 11 consecutive days. Follicular development and growth were evaluated by transvaginal sonography after six days of stimulation. Finally, ovulation was induced by a single injection (5000-10000 IU) of human chorionic gonadotropin (hCG) in women, with at least, three follicles with a diameter >18 mm. Oocytes were obtained by conventional methods as previously reported (21). Conventional IVF or ICSI was carried out in accordance with original protocols (30, 31).

Data on patients' clinical profile and treatment are shown in Table 1. This table also shows a statistically significative evaluation of the obtained values and, it is represented by p-value. These statistical values are calculated using the Kruskal Wallis H test (51), except for cause of sterility, where we used Chisquare with two degrees of freedom.

**Table 1.- Clinical profile of patients underwent controlled ovarian stimulation**

| **Parameters** | **Mean Value** | **High responders (≥12 follicles)** | **Normal responders (<12 follicles)** | **p-value** |
|---|---|---|---|---|
| n (patients) | 307 | 35 | 272 | |
| Age (years) | 32.6 ± 2.6^{¥} | 32.3 ± 2.7^{¥} | 32.7± 2.5" | 0.608^{a} |
| Cause of sterility | | | | |
| Male | 172 | 21 | 151 | |
| Tubal | 92 | 8 | 84 | 0.632^{b} |
| Both | 43 | 6 | 37 | |
| Peak of estradiol (pmol/l) | 1200 ± 932" | 2513 ± 1488^{¥} | 1030 ± 672" | 0.0001^{a} |
| Drays of stimulation | 10.8 ± 2^{¥} | 9.6 ± 1.6^{¥} | 10.5 ± 2.9^{¥} | 0.0001^{a} |
| Doses of FSH (ampoules) | 12.9 ± 6.7^{¥} | 10.7 ± 5.1^{¥} | 15.4 ± 7.5^{¥} | 0.0001^{a} |
| Production of follicles | 6.4 ± 3.6^{¥} | 14.3 ± 2.7^{¥} | 5.7 ± 2.7^{¥} | 0.0001^{a} |

| | | | | |
|---|---|---|---|---|
| ^{a} Test H Kruskall-Wallis ^{b} Chi-Square with two degrees of freedom ^{¥}Mean value ± standard deviation | | | | |

### DNA extraction

We obtained 5 ml of peripheral blood from all patients to isolate germline DNA from leukocytes. DNA extraction was performed according to standard procedures using the Nucleospin Blood Kit (Macherey-Nagel). Aliquots of DNA at a concentration of 5 ng/µl were prepared to perform polymerase chain reactions (PCRs). The rest of the stock was cryopreserved at -20°C.

### Genomic sequence

The DNA sequence used to carry out this study corresponds to the genomic sequence of the *BMP15* (bone morphogenetic protein 15) gene. The genomic sequence containing the *BMP15* gene (Genomic Contig NT_011638, locus ID 9210) was identified using the blat search at UCSC (University of California Santa Cruz, http://www.ucsc.edu).

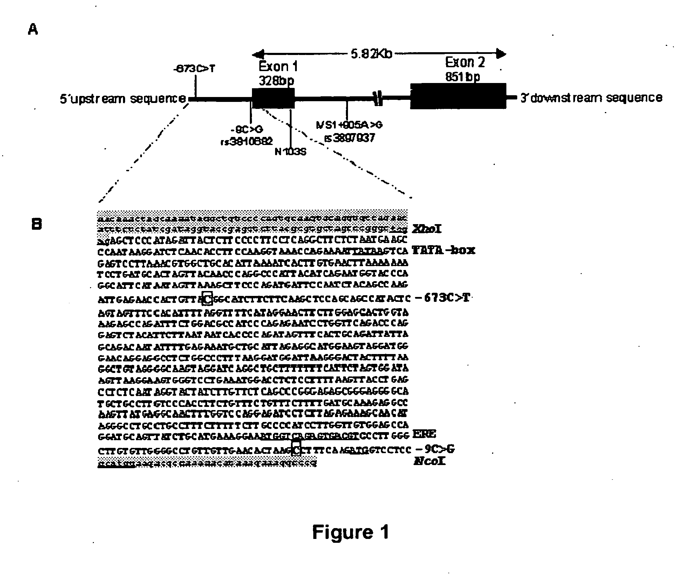

The gene is di-exonic (328 bp and 851 bp respectively), with an intronic region (4645 bp), spans 5.8 Kb and it is located at Xp11.2 (figure 1A). Information concerning any SNP identified was compared with the UCSC Genome Bioinformatics and also with Genbank Database at the National Center for Biotechnology Information (http://www.ncbi.nlm.nih.gov/).

### Genotyping of polymorphisms within the BMP15 gene using pyrosequencing technology

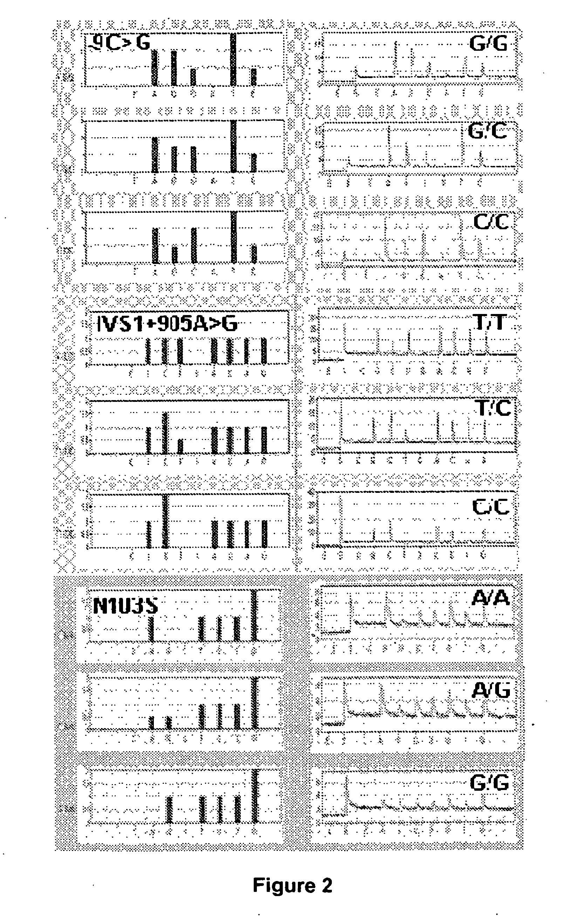

As we will discuss later, a total of 4 SNPs (single nucleotide polymorphisms) were analysed. All genotypes were carried out using the pyrosequencing technology protocols (Pyrosequencing^{™}) (32). Figure 2 shows genotype results from pyrosequencing, except for -673C>T polymorphism, due to the fact that this polymorphism is in complete linkage disequilibrium with - 9C>G and, its genotyping is not necessary (we will observe this result later). PCR assays were run on a thermal cycler machine (MJ Research, Inc. Waltman, USA) using a final volume of 20 µl. The selected primers for pyrosequencing analysis and PCR conditions are shown in table 2. The pyrosequencing machine was programmed in accordance with the manufacturer's recommendation.

**Table 2.- Amplification primer sequences and PCR conditions in pyrosequencing**

| **Localization** | **Primer** | **Sequence 5'→3'** | **PCR product size** | **PCR conditions** |
|---|---|---|---|---|
| 5'UTR (-673C>T) | PF1 | AGCTCCCATAGATTACTC (SEQ ID NO:1) | 344 | 95° 15s (54° 30s) x 50 cycles 72° 15s |
| | PR3-B | b-AGTGCTCCAAGAAGTTCC (SEQ ID NO:9) | | |
| | S4 | CAAGATTGAGAACCACTG (SEQ ID NO:10) | | |
| 5'UTR (-9C>G) | F2 | CCATCCTTGGTTGTGGAGCC (SEQ ID NO:5) | 287 | 95° 15s (66° 30s) x 50 cycles 72° 15s |
| | R2-B | b-TTCCTTGGCTGTTCGCCAGG (SEQ ID NO:11) | | |
| | S2 | GCCTGTTGTTGAACACT (SEQ ID NO:12) | | |
| Exon 1 (N103S) | F5 | CCTGGCGAACAGCCAAGGAA (SEQ ID NO:13) | 274 | 95° 15s (66° 30s) x 50 cycles 72° 15s |
| | R5-B | b-ACAAGGCAACAAAGCCTGACA (SEQ ID NO:14) | | |
| | S3 | TGAAGCCCTTGACCA (SEQ ID NO:15) | | |
| Intron 1 (IVS1+905A>G) | F-B | b-ATTGTTGCAGGACAACAGTGG (SEQ ID NO:16) | 196 | 95° 15s (62° 30s) x 50 cycles 72° 15s |
| | R | TCACAGTAAGGGTTAGGTACG (SEQ ID NO:17) | | |
| | S | CATCTCAGACTAGTGAAG (SEQ ID NO:18) | | |

### Statistical analysis

To compare allele frequencies among groups, we employed the Chi-square test with the Yates' correction or Fisher exact test using Statcalc (Epilnfo 5.1, Center for Disease Control Atlanta, GA). To compare quantitative variables among groups, mean values were analysed by using t-test for two samples or the Kruskall-Wallis test (for non-normally distributed data). For these analyses we employed Statcalc and Analysis software (Epilnfo 5.1, Centre for Disease Control, Atlanta, Georgia, USA) or Analyse-it (version 1.65; Analyse-It Software Ltd, Leeds, UK). For lineal regression studies, we used the SPSS 11.0 software.

To perform statistical analysis of genotype distribution, the test for deviation from Hardy-Weinberg equilibrium or two-point association studies, we employed tests adapted from Sasieni (33) without a Bonferroni-type correction, since the detected polymorphisms are in complete linkage disequilibrium. These calculations were performed at the online resource of the Institute for Human Genetics, Munich, Germany (http://ihg.gsf.de).

LD values between markers, haplotype construction and haplotype analysis for dichotomic traits were performed using Thesias software (http://www.genecanvas.org) based on the SEM algorithm (34). This method allows one to estimate haplotype effects by comparison to a reference (the intercept) taken here as the most frequent one. The haplotype effects are expressed like increase or decrease of the phenotypic mean with regard to reference.

Regarding the bioinformatics tools used for the sequence analysis of the 5'UTR region in *BMP15* gene, it should be stated that Translational Element Search System (TESS) software is available at http://www.cbil.upenn.edu/tess. Dragon ERE Finder software was also online consulted at http://sdmc.lit.org.sg/ERE-V2/index.

### Example 1.- Genotype analysis of the mutations within BMP15 gene in the study population.

Automated DNA sequencing methods were employed to scan the entire coding region of the *BMP15* gene in selected specimens. Overlapping PCRs cowering the coding sequence of gene were designed. PCR products were purified and bi-directionally sequenced using the corresponding primers shown in table 3. Sequencing reactions were performed using the CEQ Dye Terminator Cycle Sequencing Quick Start Kit (Beckman Coulter, Inc), according to the manufacturer's instructions, and analysed using CEQ^{™} 8000 Genetic Analysis System (Beckman Coulter, Inc., Fullerton, CA)

**Table 3.- Amplification primer sequences and PCR conditions in sequencing of BMP15 gene**

| **Localization** | **Primer** | **Sequence 5'→3'** | **PCR product size** | **PCR conditions** |
|---|---|---|---|---|
| 5'UTR | PF1 | AGCTCCCATAGATTACTC (SEQ ID NO:1) | 773 | 95° 15s (54° 30s) x 50 cycles 72° 15s |
| | PR1 | CTGGACCAAAGTTGCCT (SEQ ID NO:2) | | |
| 5'UTR | PF2 | GGTACTATCTTGTTCTCAGC (SEQ ID NO:3) | 456 | 95° 15s (66° 30s) x 50 cycles 72° 15s |
| | R2 | TTCCTTGGCTGTTCGCCAGG (SEQ ID NO:4) | | |
| Exon 1 | F2 | CCATCCTTGGTTGTGGAGCC (SEQ ID NO:5) | 541 | 95° 15s (66° 30s) x 50 cycles 72° 15s |
| | R5 | ACAAGGCAACAAAGCCTGACA (SEQ ID NO:6) | | |
| Exon2 | e2F | GAGGTAAGAAGCTAAACCTC (SEQ ID NO:7) | 983 | 95° 15s (66° 30s) x 50 cycles 72° 15s |
| | e2R | TACAGGATTACTTGCAGCTT (SEQ ID NO:8) | | |

Initially, we released a preliminary association study dividing patients in poor (≤3 follicles), high (≥2 follicles) and normal (between 4 and 11 follicles) responders. In these patients, we analysed the genotype distribution of two selected polymorphisms, located in the 5'UTR region, identified as -9C>G (rs3810682), and in intron 1, identified as IVS1 +905A>G locus (rs3897937), of the *BMP15* gene. Preliminary statistical results indicated that high responder patients have a different allelic frequency and genotype distribution in both markers of the *BMP15* gene, when compared to normal responders. In contrast, poor responders do not display any significant genetic association compared with normal responders in both markers.

Therefore, we focused our studies only on patients with a high follicle production and, rFSH treated women were divided into two groups, patients producing ≥12 follicles (called high responders again) and patients producing <12 follicles (group together and called normal responders from now). The clinical profile in Table 1 corresponds to these groups.

The repeated Chi-squared tests adapted from Sasieni (table 4) showed a Minimum p-value, for risk allele G in both markers, applying the homozygous test (p = 0.0001 for -9C>G and p = 0.004 for IVS1 +905A>G). These statistical analyses point to the existence of a functional DNA variant close to the *BMP15* locus, affecting follicle production. For this reason, we sequenced the entire gene looking for functional variants.

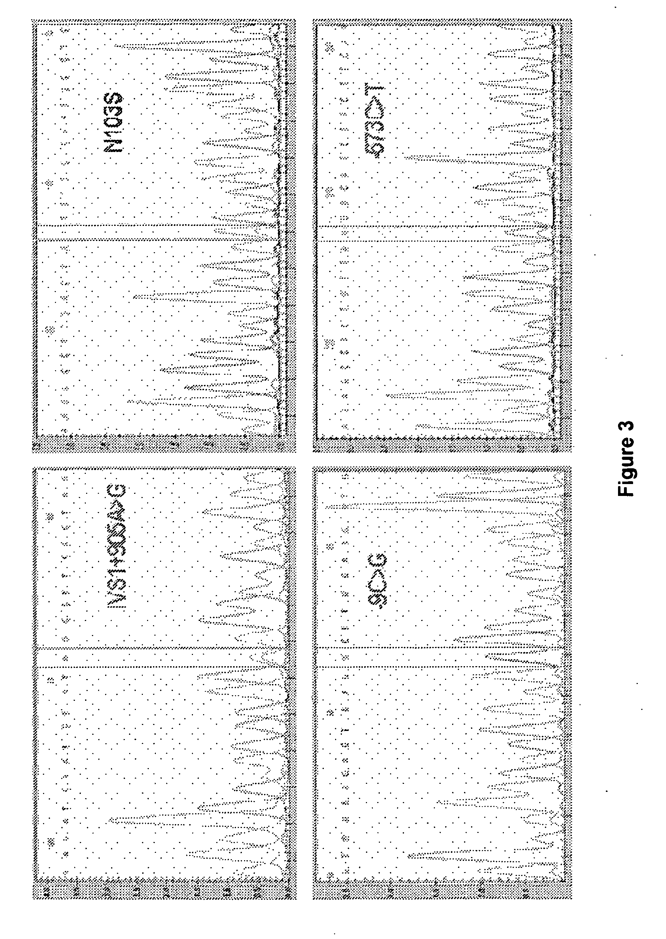

Looking for a functional mutation within *BMP15* gene, we determined the complete coding sequence in 35 high responders, using bi-directional automated capillary DNA sequencing. We identified two new single nucleotide DNA variants. The first one appeared again in the 5'UTR region, and it was identified as -673C>T. The other mutation appeared in the exon 1, c.308A>G locus, and it resulted in a missense mutation in codon 103 of the protein (N103S). Figure 3, at the right side, shows the sequence chromatographs of the *BMP15* gene regions containing above mentioned DNA polymorphisms (N103S upper right image and, -673C>T lower right image), whereas at the left side, it shows the sequence chromatographs containing the known polymorphisms (IVS1+905A>G upper left image and, -9C>G lower left image). However, the Y235C nonconservative mutation described by Di Pasquale et al. (45) was not identified in the study population.

Using these new variants, we again investigated their allelic frequency and genotype distribution in 307 patients undergoing COS. These results are shown in Table 4 and (as we previously commented), they correspond to test adapted from Sasieni (33).

**Table 4.- Clinical profile of high responders versus no normal responders undergoing controlled ovarian stimulation**

| **Mutation** | **n (patients)** | **High responders (≥12 follicles)** | **Normal responders (<12 follicles)** | **p-Values** |
|---|---|---|---|---|
| | | | | 0.72/0.12 (a) |
| -9C>G^{£} | | | | 0.003 (b) |
| (CC/CG/GG) | 172/120/15 | 14/15/6 | 158/105/9 | 0.22 (c) |
| -673C>T^{£} | | | | **0.0001** (d) |
| (CC/CT/TT) | | | | 0.04 (e) |
| | | | | 0.0025 (f) |
| | | | | 1/0.19 (a) |
| | | | | 0.014 (b) |
| IVS1+905A>G | 140/141/26 | 11/17/7 | 129/124/19 | 0.23 (c) |
| (AA/AG/GG) | | | | **0.004** (d) |
| | | | | 0.079 (e) |
| | | | | 0.011 (f) |
| | | | | 1/0.52 (a) |
| | | | | 0.26 (b) |
| N103S | 274/32/1 | 29/6/0 | 245/26/1 | 0.16 (c) |
| (AA/AG/GG) | | | | 0.73 (d) |
| | | | | 0.19(e) |
| | | | | 0.23 (f) |

| | | | | |
|---|---|---|---|---|
| ^{£} -673C>T and -9C>G loci are a sintenic variant with the same allelic frequencies and in complete linkage disequilibrium (LD). All the calculations have been done for the allele 2 in each marker, the least frequent: G, T, G and S alleles for -9C>G- -673C>T, IVS1+905A>G and N103S markers respectively. (a) Deviation from Hardy-Weinberg equilibrium in cases/controls of patients (b) Allele frequency differences test. (c) Heterozygous test. (d) Homozygous test. (e) Allele positivity test. (f) Armitage's trend test. | | | | |

As shown in this table, concerning the two known markers, the allelic frequencies in our sample are 0.76 for C allele and 0.24 for G allele in 5'UTR - 9C>G locus (rs3810682) and, 0.69 for A allele and 0.31 for G allele in intron 1 IVS1+905A>G locus (rs3897937). The observed heterozygosity was 0.39 and 0.46 respectively. We observed that the genotype frequencies were in accordance with the Hardy-Weinberg Equilibrium Law (p > 0.68).

With respect to new detected mutations, allelic frequencies in our patients were 0.76 for C allele and 0.24 for T allele in the -673C>T polymorphism and, the observed heterozygosity for the locus was 0.39 (results similar to the -9C>G locus). In the N103S locus the allelic frequencies were 0.94 for allele A and 0.06 for allele G and, the observed heterozygosity for this locus was 0.10. The genotype frequencies are in accordance with the Hardy-Weinberg Equilibrium Law (p > 0.52). N103S variant does not show a different genotype distribution when we compare high responders versus the rest of patients (p > 0.16). For this reason, we think that this variant within *BMP15* locus is not involved in FSH over-response. Although, as N103S is in complete linkage disequilibrium with risk haplotype detected in this study (see below), it could be possible that this missense mutation explained the association to FSH over-response, partially detected in this study. Due to these results and, to the elevated association of 5'UTR markers principally, we decided to re-construct haplotypes of the *BMP15* gene using the four validated genetic variants and so, to look for risk haplotypes associated with high response to FSH.

### Example 2.- Haplotype analysis in the pharmacogenetic assay

In order to analyse the degree to which these polymorphisms are in linkage disequilibrium (LD), we performed standardized pairwise LD (D') using Thesias software. The value D' of ± 1 indicates complete LD, positive when the rare allele of each polymorphism is preferentially associated and, negative when the rare and frequent alleles are associated (35). Thus, we verified that - 673C>T locus is a sintenic variant of the -9C>G locus, with the same allelic frequencies and in complete LD. Besides, the four polymorphisms are in complete LD between them (D' > 0.95 and p < 0.004).

Using Thesias software, we reconstructed haplotypes and calculated their frequencies in 307 women. Rare haplotypes with minor-allele frequencies of less than 1% were excluded. The program identified four distinct haplotypes in our population, resulting from combination and permutation of each individual marker in this study (TGGA, TGGG, CCGA and CCAA). Table 5 shows the obtained results. Moreover, this table shows the number of chromosomes where we detected each haplotype in both groups of patients (normal or high responders), the p value for statistical significance and the Odds Ratio (OR), together with its deduced confidence interval (CI) in square brackets. OR values show the probability of suffering an illness in patients carrying a specific allele: an OR>1 value indicates a predisposition to suffer an illness, whereas an OR<1 value indicates a protection from illness (lower probability to suffer it).

**Table 5.- Comparison of BMP15 haplotype frequencies**

| **Haplotype** | **Normal responders Chromosome** | **%** | **High responders Chromosome** | **%** | **p-value** | **OR [CI]** |
|---|---|---|---|---|---|---|
| I TGGA | 96 | 17.6 | 21 | 30 | 0.0044 | 2.4 [1.31-4.26] |
| II TGGG | 27 | 5 | 6 | 8.6 | 0.1071 | 2.3 [0.84-6.22] |
| III CCGA | 38 | 7 | 4 | 5.7 | 0.8282 | 1.13 [0.37-3.51] |
| IV CCAA | 382 | 70.2 | 39 | 55.7 | 0.0056 | 0.43 [0.24-0.78] |
| Total | 543 | 99.8 | 70 | 100 | 0.037 (a) | |

| | | | | | | |
|---|---|---|---|---|---|---|
| (a) The global haplotypic effect using Standard Pearson Chi-square with three degrees of freedom. | | | | | | |

In this study, we demonstrated that haplotype TGGA is over-represented in high responders when compared to no high responders, 30% versus 17.6% (p = 0.0044, OR = 2.36). The global haplotypic effect is calculated using likelihood ratio test (LRT) with three degrees of freedom (p = 0.037) (table 5). Intriguingly, the haplotype carrying N103S aminoacidic change (haplotype II, TGGG) has almost identical OR to TGGA haplotype. Suggesting that i) TGG(G/A) haplotype (including TGGG and TGGA alleles) carries a functional common variant or, ii) N103S is a functional variant although there are other non coding variants involved to over-response.

When we analyzed the haplotypic background in each polymorphic loci, we did not observe any genetic statistical significance (p >0.8). This result is in accordance with the two-point association studies carried out to each individual genetic marker. Only two haplotypes, TGG- and CCA- for -673C>T, -9C>G and IVS1+905A>G polymorphisms respectively, were statistically significant. Haplotype TGG- is a risk allele, whereas haplotype CCA- is a protective allele, for women in COS over-response.

We found no associations with any haplotype in the QTL analysis of age, peak oestradiol or follicle production, although, a trend towards association was obtained for peak oestradiol in haplotype TGGA (n = 298, p = 0.083). This result is completely compatible with the association involved in rFSH over-response.

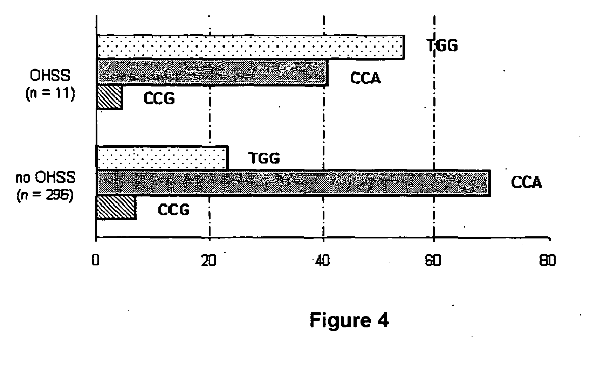

Furthermore, we separated the patients with early symptoms of OHSS (presence of the early sign symptoms or biochemical data: enlarged ovaries associated with pelvic pain and, if the stimulation treatment continues, it could cause a complete syndrome), due to the fact that it is the hardest end-point of FSH over-response. When we compared them with the rest of patients (no OHSS), we observed a strong positive association with TGG- haplotype (p = 0.0006, OR = 4.7) and CCA- (p = 0.0006, OR = 0.2). As shown in the figure 4 (the haplotype frequencies, in percentage, in patients with early symptoms of OHSS versus rest of patients) and in the resume of the associated statistical parameters present in the table 6, both haplotype frequencies were inverted in OHSS patients, with a significant global haplotypic effect on the *BMP15* gene (p = 0.0054). Nevertheless, they should highlight that the N103S variant itself is nominally associated with OHSS risk independently (p = 0.05). This shows that this aminoacidic sequence variation could partially explain the results observed in table 6. Although, it also reveals the existence of, at least, another functional variant linked to TGGA haplotype.

**Table 6.- Relation of the haplotypic context in patients with early symptoms of OHSS**

| **Haplotype** | **p-value** | **OR [CI]** |
|---|---|---|
| CCG- | 0.8376 | 1.3 [0.1 -12.3] |
| CCA- | 0.0006 | 0.2 [0.09 - 0.5] |
| TGG-* | 0.0006 | 4.7 [1.9 - 11.5] |

| | | |
|---|---|---|
| Global chi-square = 10.43 (2 degrees of freedom) and global p-value = 0.0054. *TGG-haplotype including TGGG and TGGA (N103S detected during the sequencing) haplotypes. | | |

All these results indicate that, apart from N103S, might exist a non coding mutation, related to rFSH over-response phenotype, close to *BMP15.* For this reason we decided to perform a functional characterisation of the described haplotypes in the 5'UTR region and to analyze the *BMP15* gene expression.

### Example 3.- Functional studies of BMP15

In order to perform a functional characterisation of the sequence under study, we cloned 1 Kb up-stream of the coding *BMP15* gene, together with, the initial nucleotides in the coding sequence. The amplified region sequence is shown in SEQ ID NO:19. The 5'UTR region of *BMP15* was amplified to a final volume of 10 µl containing 10 ng of genomic DNA, 0.2 µM each amplification primer, 1.5 mM MgCl₂, 75 µM dNTP and 1U of High fidelity Taq Polymerase (Roche Diagnostics, Germany). To perform these reactions, we employed DNA of homozygous patients for CCAA and TGGA haplotypes. For this reason, the amplified fragments have both SNPs located in 5'UTR region within *BMP15* gene (-673C>G and -9C>G). The primers employed are:
Forward:
   Xhol-F GAGCTCGAGAGCTCCCATAGATTACTC (SEQ ID NO:19)
Reverse:
   NcoI-R GGCCATGGGGAGGACCATCTTGAAAG (SEQ ID NO:20)

These primers include Xhol and NcoI restriction sites underline in Xhol-F and Ncol-R respectively.

PCR amplification conditions were: an initial denaturation step of 95°C for 30 seconds, followed by 50 cycles of 62°C for 30 seconds and, a final step of 72°C for 60 seconds.

Amplicons were purified, digested and cloned into multiple clonation site (MCS) of pGL3-Basic vector (Promega, USA). Thanks to the design of the primers, the resulting amplification contained the four initial amino acids of BMP15 and, they were cloned in-frame to the initial methionine of the firefly Luciferase. Thus, both amplicones (CC and TG) were cloned in-frame to the N-terminal domain of the luciferase. The characteristics of the pGL3-Basic plasmid using as vector, together with other used vectors in this example are shown in the table 7.

**Table 7.- Plasmid used in the functional study of the 5'UTR region**

| **Name** | **Description** | **Reference** |
|---|---|---|
| pGL3-Basic | ColE1. Amp^{R}. 4.8 Kb. Promoter-less vector with the Firefly luciferase gene down-stream a multi cloning site. | Promega |
| pGL3-Control | ColE1. Amp^{R}. 5.25 Kb. Vector expressing the Firefly luciferase gene by mean of the SV40 promoter and the SV40 enhancer. | Promega |
| pGL3-enhancer | ColE1 . Amp^{R}. 5.5 Kb. PGL-3 basic and the SV40 enhancer. | This study |
| pRL-null | ColE1. Amp^{R}. 3.3 Kb. Promoter-less vector with leaky expression of the Renilla luciferase gene. | Promega |
| pCC29 | ColE1. Amp^{R}. 5.9 Kb. pGL3-Basic derived vector containing the CC 5'UTR of *BMP15* cloned in XhoI/NcoI. | This study |
| pTG9 | ColE1. Amp^{R}. 5.9 Kb. pGL3-Basic derived vector containing the TG 5'UTR of *BMP15* cloned in XhoI/NcoI. | This study |
| pTC2 | ColE1. Amp^{R}. 5.9 Kb. pGL3-Basic derived vector containing the TC 5'UTR of *BMP15* cloned in XhoI/NcoI. | This study |
| pCG15 | ColE1. Amp^{R}. 5.9 Kb. pGL3-Basic derived vector containing the CG 5'UTR of *BMP15* cloned in XhoI/NcoI. | This study |
| pECC2 | ColE1. Amp^{R}. 6.15. pCC29 derived vector with the SV40 enhancer cloned down-stream the luciferase gene. | This study |
| pETG2 | ColE1. Amp^{R}. 6.15. pTG9 derived vector with the SV40 enhancer cloned down-stream the luciferase gene. | This study |
| pETC2 | ColE1. Amp^{R}. 6.15. pTC2 derived vector with the SV40 enhancer cloned down-stream the luciferase gene. | This study |
| pECG2 | ColE1. Amp^{R}. 6.15. pCG15 derived vector with the SV40 enhancer cloned down-stream the luciferase gene. | This study |

Ligation reactions were performed using a rapid ligation kit (New England Biolabs, USA) according to the manufacturer's instructions. Plasmids were then chemically transformed to XL1-Blue competent cells. Colonies were checked for the presence of the insert and positive constructs were sequenced to discard the constructs bearing point mutations incorporated during the PCR. As a result, we obtained pTG9 vector for studying the mutant haplotype and, pCC43 as a wild type vector.

The resulting vectors contained either the wild type or the mutant haplotypes, followed by a fusion protein containing the four initial amino acids of BMP15 and, next the Luciferase. The rationale for this approach is that, -9C>G polymorphism is located close to the Kozak sequence of the *BMP15* gene. This led us to postulate that this polymorphism could be involved in a change of the 5'UTR region conformation, therefore affecting the ribosome recognition. This might lead to an altered translational efficiency (36, 37). For this reason, a translational fusion to the initial amino acids of the reporter gene (Luciferase) was required. Indeed, in order to determine if the putative effect was due to the first, the second or an additive effect of both polymorphisms, DNA shuffling between the wild type and the mutant haplotypes was carried out. In these additional vectors, the two variations were isolated and completed with the corresponding wild type sequence (pTC2 and pCG15). Therefore, four constructs were initially tested and their luciferase activity was compared to the promoter-less vector.

To carry out this study, we transfected different cell lines and, they were cultured in Dulbecco's modified Eagle's medium (DMEM) or MEM supplemented with 20% fetal calf serum at 37°C in 95% humidified air and 5% CO₂. Luciferase activity was determined after a 24 and 48 hour incubation. In order to test different genetic backgrounds, four cell lines were included in this study: Du145, MCF-7, K562 and GH3. Transient transfection protocols were optimized from each cell line, and internal controls allowed us to assure transfection efficiencies between 25-80%. pRL-null vector was included for internal normalization; allowing inter- and intra-experimental comparison.

After three independent experiments, we concluded that no expression differences could be detected among the wild type and mutant constructs. In addition, luciferase activities were similar to those obtained with the pGL3-basic vector (promoter-less control). This led us to propose that specific activation signals may be required for triggering expression from our vectors in transfected cells.

A detailed *in silico* analysis of the *BMP15* 5'UTR region using both, TESS and Dragon ERE Finder software programs, highlighted an oestrogen receptor element (ERE) located 80 bp up-stream from the initial ATG (figure 1B). For this reason, we stimulated transfected MCF-7 cells with 5 nM of 17-alpha ethinylestradiol (38). Luciferase activity was measured at 0h, 0.5h, 1h, 4h, 8h and 24h post-induction. No differences were observed between constructs bearing the wild type and mutant haplotypes. Moreover, no up-regulation was detected during any experiment.

We also considered the possibility that the ERE was exerting a negative effect on promoter activity. This regulatory effect may be due to the oestrogenic activity of the phenol-red from the culture media (39). In order to eliminate this possibility, transfected MCF-7 was cultured in a red-free medium, with identical results.

Previous studies in mice linked *bmp15* expression via stimulation with FSH. Therefore, we looked for differential promoter activities of the candidate plasmids under this condition. After transfection, Du145 were cultured in serum-free media with/without 0.1 U/mL rFSH (Sigma-Aldrich, Missouri, USA). As for the above-mentioned experiments, luciferase expression was measured following a time-course kinetic. Again, no induction was observed upon stimulation.

Some authors have described the negative feedback circuit linking the oocyte and the granulosa cells, involving Kit Ligand (40). We therefore proposed that by stimulating with Kit Ligand we might modulate *BMP15* promoter expression. For this purpose, we electroporated the K562 cell line, which is known to express an active Kit Receptor (41). Identical kinetic analyses were performed and neither promoter activity nor differences between constructs were observed.

Finally, each kinetic analysis was repeated using the plasmids containing the SV40 enhancer down-stream of the *Luciferase* gene (pECC, pETG, and the corresponding controls). For that reason, pGL3-control was digested with *BamH*I/*Xba*I and a 0.3 Kb fragment containing the SV40 enhancer was isolated. This sequence was cloned down-stream of the *Luciferase* gene of pCC43, pTG9, pTC2, pCG15 and pGL3-basic vectors, thus generating pECC, pETG, pETC, pEGG and pGL3-enhancer respectively. Clones were sequenced and prepared for cell culture transfection. The achieved results in the constructs did not show any difference in the promoter activity.

Functional studies described in this example did not prove that a functional mutation in *BMP15* 5'UTR region exists. It is possible that our approach is not suitable for assessing the function, due to the lack of expression outside of oocyte cells. A possible reason for this is that an additional sequence up- and/or down-stream would be necessary for transcription initiation. In addition, tissue-specific transcription factors present in the oocyte might be required for BMP15 expression. Moreover, we must consider the possibility that multiple effects might be necessary for triggering BMP15 expression (gene silenced in other tissues due to specificity of oocyte). In fact, in a further *in silico* characterisation of the *BMP15* 5'UTR region, the applicants found multiple silencer transcription factors such as YY1 and GR (glucocorticoid receptor).

In conclusion, the present invention identifies the TGG- allele within *BMP15* gene involvement in FSH over-response and OHSS.

Finally, it would be possible that the association observed (p = 0.0001) was caused by chance due to small sample size (n = 35). For that reason, we decided to analyze *BMP15* gene in other independent population and to confirm the association of *BMP15* haplotype with alterations in the normal function of the ovary. We will describe this analyze in the next study.

### Association study in postmenopausal women

Taking into account the results of the pharmacogenetics study, we investigated the possibility that *BMP15* gene and developed markers could have an important role, in the oocyte recruitment during a menstrual cycle in women without hormonal treatment. The principal idea of the study is to analyze the variance in age at menopause in women, with a menopause due to natural causes. As we previously commented, our working hypothesis is based on that, if *BMP15* allele contributes to the follicular recruitment induced by FSH really, women carrying these alleles could consume a high number of follicles per cycle in a natural way and, her ovarian reserve could get exhausted ahead of time. As it is well known, an early exhaustion of follicular reserve is the principal cause of menopause in women and, *BMP15* could be a predictor of the early exhaustion in the ovarian reserve. Therefore, factors to accelerate or decelerate the follicular consume per cycle, could have a predictive utility to identify women with risk to present an early menopause or a premature ovarian failure. Moreover, these factors could present a new strategic molecular way for therapeutic interventions that allow to control, to accelerate or to decelerate the appearance of menopause. For this reason, we carried out the described study in the example 4.

### Example 4.- Association study with early menopause, premature ovarian failure, age at menopause and reproductive periods

### Patients

During this work, we used 750 Spanish postmenopausal women with an extensive clinical and epidemiological analysis and, a Bone Mass Density (BMD) measurement using Norland or Holigic (QDR/1000W) densitometers. We standardized BMD values using an international reference table and, we employed these data to calculate the lumbar T-score. This data is based in a densitometric value that represents the number of standard deviations (SD), that the subject stands apart in relation to the average of the values in women of 25 years old. It is considered that a normal T-score value over -1SD is not indicative of osteopenia or osteoporosis, whereas a value under -1SD (over in absolute value) is indicative of osteopenia or different grades of osteoporosis. Parameters and criterions used in this study are:
a. Osteoporosis: Lumbar T-score under -2.5 SD and/or osteoporotic fractures.
b. Age at menopause: physiological cessation of menstrual cycles during, at least, three months.
c. Early menopause: appearance of menopause between 40 and 45 years of age.
d. Premature ovarian failure: appearance of menopause before 40 years of age.
e. Reproductive period= Age at menopause-Age at menarche

Excluded patients for this study are:
Women with previous antiresorptive treatment. Women with a surgical induced menopause were analyzed in an independent manner and, they were excluded from age at natural menopause studies. After filtering valid cases, we selected 553 women with a natural menopause without a resorptive treatment.

### Genotyping study

This study is similar to the analysis described in Examples 1 and 2 for the predisposition to develop OHSS, analyzing the presence of the same SNP (used in these examples) in the new study population. The obtained statistical results are shown in table 8.

**Table 8.- Relation of -9C>G and IVS1+905A>G markers with premature ovarian failure, early menopause, osteoporosis and cuantitative parameters, in 553 Spanish postmenopausal women.**

| **PREMATURE OVARIAN FAILURE (POF)** | | | | |
|---|---|---|---|---|
| (27 POF women y 526 non POF women) | | | | |
| **Marker** | **Test** | **p-value** | **OR [CI]** | **Comment** |
| -9C>G | Not associated | Not significant | Not associated | Not associated |
| IVS1+905 A>G | Heterozygous | 0.05 | 2.182 | Show a high risk in heterozygous women |
| | | | [0.981-4.853] | |
| IVS1+905 A>G AG vs Rest | Pearson | 0.032 | 2.3 | Show a high risk in heterozygous women |

| **EARLY MENOPAUSE (EM)** | | | | |
|---|---|---|---|---|
| (87 EM women y 437 non EM women) | | | | |
| **Marker** | **Test** | **p-value** | **OR [CI]** | **Comment** |
| -9C>G | Armitage | 0.04 | 1.47 | Weak association |
| IVS1+905 A>G | Heterozygous | 0.02 | 1.710 | Show a high risk in heterozygous women |
| | | | [1.052-2.778] | |
| IVS1+905 A>G AG vs Rest | Pearson | 0.03 | 1.63 | Show a high risk in heterozygous women |

| **OSTEOPOROSIS** | | | | |
|---|---|---|---|---|
| (171 patients with osteoporosis y 363 controls) | | | | |
| **Marker** | **Test** | **p-value** | **OR [CI]** | **Comment** |
| -9C>G | Not associated | Not significant | Not associated | Not associated |
| IVS1+905 A>G | Homozygous | 0.04 | 1.968 | Weak |
| | | | [1.030-3.763] | association |
| IVS1+905 A>G AG vs Rest | Pearson | Not significant | Not associated | Not associated |

| **CUANTITATIVE ANALYSIS OF THE STUDY PARAMETERS** | | | | |
|---|---|---|---|---|
| **Genotypes** | **Age at menopause (mean)** | **Reproductive period (mean)** | **T-score (mean)** | **Test and p-value** |
| -9C>G | | | | ANOVA. Not significant in any cases, p>0.27 |
| GG | 48.5 | 35.7 | -1.74 | |
| GC | 47.9 | 34.9 | -1.77 | |
| CC | 48.6 | 35.5 | -2.07 | |
| IVS1+905 A>G | | | | ANOVA. Significative for age at menopause (p=0.03)* and reproductive period (p=0.029) |
| AA | 48.8 | 35.9 | -1.74 | |
| AG | 47.5 | 34.6 | -1.74 | |
| GG | 48.3 | 35.9 | -1.98 | |
| IVS1+905 A>G (Heterozygous vs rest) | | | | T Student. Significative for age at menopause (p=0.01)** and reproductive period (p=0.025) |
| AG | 47.5 | 34.7 | -1.74 | |
| Rest | 48.8 | 35.9 | -1.77 | |

| | | | | |
|---|---|---|---|---|
| *In this case, we used the Kruskall Wallis tests because we observed a difference in variance in age at menopause between groups. ** In this case, we used the Mann-Whitney U tests because we observed a difference in variance in age at menopause between groups. | | | | |

In the *BMP15* genotyping study of postmenopausal women, the maximum effect was detected in the IVS1+905A>G marker, when we compared heterozygous women versus the rest. This marker is associated with early menopause (p=0.006) and, premature ovarian failure (p=0.028). Moreover, this marker is also associated with age at menopause, since heterozygous women for this marker show a mean of age at menopause 1.29 years before than rest of women (p=0.004), and reproductive period, with a similar reduction of 1.19 years (p=0.008).

Regression analysis, employing general lineal model (GLM) in SPSS software and, using age at menopause as independent variable, showed that gene effect in age at menopause is completely independent to other known factors (influential factors of this physiological parameter), like age, T-score, parity, smoke habit, etc (p=0.005) (table 9).

**Table 9.- Lineal model to verify the independent effect of the heterozygous IVS1+905A>G marker with regard to other risk well-known factors that affect to age at menopause.**

| **Independent variables** | **Degrees of freedom** | **F** | **p-value** |
|---|---|---|---|
| Adjusted model | 8 | 6.086 | p<0.0001 |
| Intersection | 1 | 23.6555 | p<0.0001 |
| Lumbar T-score | 1 | 10.36 | p=0.001 |
| Age | 1 | 16.231 | p<0.0001 |
| Smoke habit | 1 | 2.737 | p=0.099 |
| Pregnancies | 1 | 0.84 | p=0.360 |
| Miscarriages | 1 | 2.237 | 0.135 |
| Preterm | 1 | 0.412 | 0.521 |
| Parity | 1 | 4.76 | 0.03 |
| IVS1+905 A>G | 1 | 7.847 | 0.005 |

| | | | |
|---|---|---|---|
| R²: 0.088 (adjusted R² = 0.073). R represents the percentage of age at menopause variance which can be explained by this group of variables. Individual contribution of each variable can be estimated similarly. | | | |

This result confirms the previous results and identifies IVS1 +905A>G variant within *BMP15* gene like a molecular predictor and, moreover, it is completely independent to predict age at menopause in women. This result leaves open the possibility of therapeutic interventions in this level to modify or to correct the physiological parameters influenced by *BMP15* gene. Furthermore, it could be possible to employ this marker as a diagnostic method to predict the risk of early menopause and premature ovarian failure.

### References

1. Salha O, Balen AH. New concepts in superovulation strategies for assisted conception treatments. Curr Opin Obstet Gynecol 2000;12:201-6
2. Kligman I, Rosenwaks Z: Differentiating clinical profiles: predicting good responders, poor responders, and hyperresponders. Fertil. Steril 2001;76:1185-90
3. Smitz J, Camus M, Devroey P. Incidence of severe ovarian hyperstimulation Syndrome after GnRH agonist/HMG superovulation for in vitro fertilization. Human Reprod 1990;5:933-40
4. Delvigne A, Rozenberg S. Review of clinical course and treatment of ovarian hyperstimulation syndrome (OHSS). Hum Reprod 2003;9:77-96
5. Navot D, Relou A, Birkenfeld A, Rabinowitz R, Brzezinski A, Margalioth EJ. Risk factors and prognostic variables in the ovarian hyperstimulation syndrome. Am J Obstet Gynecol 1988;159:210-5
6. Navot D, Margalioth EJ, Laufer N, Birkenfeld A, Relou A, Rosler A, Schenker JG. Direct correlation between plasma renin activity and severity of the ovarian hyperstimulation syndrome. Fertil Steril 1987;48:57-61
7. Morris RS, Paulson RJ. Increased angiotensin-converting enzyme activity in a patient with severe ovarian hyperstimulation syndrome. Fertil Steril 1999;71:562-3
8. Levin ER, Rosen GF, Cassidenti DL, Yee B, Meldrum D, Wisot A, Pedram A. Role of vascular endothelial cell growth factor in Ovarian Hyperstimulation Syndrome. J Clin Invest. 1998;102:1978-85
9. Chen CD, Chen HF, Lu HF, Chen SU, Ho HN, Yang YS. Value of serum and follicular fluid cytokine profile in the prediction of moderate to severe ovarian hyperstimulation syndrome. Hum Reprod 2000;15:1037-42
10. Barak V, Elchalal U, Edelstein M, Kalickman I, Lewin A, Abramov Y. Interleukin-18 levels correlate with severe ovarian hyperstimulation syndrome. Fertil Steril 2004;82:415-20
11. Enskog A, Nilsson L, Brannstrom M. Peripheral blood concentrations of inhibin B are elevated during gonadotrophin stimulation in patients who later develop ovarian OHSS and inhibin A concentrations are elevated after OHSS onset. Hum Reprod 2000;15:532-8
12. Daniel Y, Geva E, Amit A, Eshed-Englender T, Bar-Am A, Lessing JB. Soluble endothelial and platelet selectins in serum and ascitic fluid of women with ovarian hyperstimulation syndrome. Am J Reprod Immunol 2001;45:154-60.
13. Mozes M, Bogokowsky H, Antebi E, Lunenfeld B, Rabau E, Serr DM, David A, Salomy M. Thromboembolic phenomena after ovarian stimulation with human gonadotrophins. Lancet 1965;2:1213-5
14. Cluroe AD, Synek BJ. A fatal case of ovarian hyperstimulation syndrome with cerebral infarction. Pathology 1995;27:344-6
15. Beerendonk CC, van Dop PA, Braat DD, Merkus JM. Ovarian hyperstimulation syndrome: facts and fallacies. Obstet Gynecol Surv 1998; 53:439-49
16. Semba S, Moriya T, Youssef EM, Sasano H. An autopsy case of ovarian hyperstimulation syndrome with massive pulmonary edema and pleural effusion. Pathol Int 2000;50:549-52
17. Georgiou I, Konstantelli M, Syrrou M, Messinis IE, Lolis DE. Oestrogen receptor gene polymorphisms and ovarian stimulation for in-vitro fertilization. Hum Reprod 1997;12:1430-3
18. Sundarrajan C, Liao WX, Roy AC, Ng SC. Association between estrogen receptor-beta gene polymorphisms and ovulatory dysfunctions in patients with menstrual disorders. J Clin Endocrinol Metab 2001;86:135-9
19. Perez Mayorga M, Gromoll J, Behre HM, Gassner C, Nieschlag E, Simoni M. Ovarian response to follicle-stimulating hormone (FSH) stimulation depends on the FSH receptor genotype. J Clin Endocrinol Metab 2000;85:3365-9
20. Sudo S, Kudo M, Wada S, Sato O, Hsueh AJ, Fujimoto S. Genetic and functional analyses of polymorphisms in the human FSH receptor gene. Mol Hum Reprod 2002;8:893-9
21. de Castro F, Ruiz R, Montoro L, Perez-Hernandez D, Sanchez-Casas Padilla E, Real LM, Ruiz A. Role of follicle-stimulating hormone receptor Ser680Asn polymorphism in the efficacy of follicle-stimulating hormone. Fertil Steril 2003;80:571-6
22. de Castro F, Moron FJ, Montoro L, Galan JJ, Hernandez DP, Padilla ES, Ramirez-Lorca R, Real LM, Ruiz A. Human controlled ovarian hyperstimulation outcome is a polygenic trait. Pharmacogenetics 2004;14:285-93
23. Daelemans C, Smits G, de Maertelaer V, Costagliola S, Englert Y, Vassart G, Delbaere A. Prediction of severity of symptoms in iatrogenic ovarian hyperstimulation syndrome by follicle-stimulating hormone receptor Ser680Asn polymorphism. J Clin Endocrinol Metab 2004;89:6310-5
24. Smits G, Olatunbosun O, Delbaere A, Pierson R, Vassart G, Costagliola S. Ovarian hyperstimulation syndrome due to a mutation in the follicle-stimulating hormone receptor. N Engl J Med 2003;349:760-6
25. Vasseur C, Rodien P, Beau I, Desroches A, Gerard C, de Poncheville L, Chaplot S, Savagner F, Croue A, Mathieu E, Lahlou N, Descamps P, Misrahi M. A chorionic gonadotropin-sensitive mutation in the follicle-stimulating hormone receptor as a cause of familial gestational spontaneous ovarian hyperstimulation syndrome. N Engl J Med 2003;349:753-9
26. Montanelli L, Delbaere A, Di Carlo C, Nappi C, Smits G, Vassart G, Costagliola S. A mutation in the follicle-stimulating hormone receptor as a cause of familial spontaneous ovarian hyperstimulation syndrome. J Clin Endocrinol Metab 2004;89:1255-8
27. Galloway SM, McNatty KP, Cambridge LM, Laitinen MP, Juengel JL, Jokiranta TS, McLaren RJ, Luiro K, Dodds KG, Montgomery GW, Beattie AE, Davis GH, Ritvos O. Mutations in an oocyte-derived growth factor gene (BMP15) cause increased ovulation rate and infertility in a dosage-sensitive manner. Nat Genet 2000;25:279-83
28. Chang H, Brown CW, Matzuk MM. Genetic analysis of the mammalian transforming growth factor-beta superfamily. Endocr Rev 2002;23:787-823
29. Wikland M, Borg J, Hamberger L, Svalander P. Simplification of IVF: minimal monitoring and the use of subcutaneous highly purified FSH administration for ovulation induction. Hum Reprod 1994;9:1430-6
30. Lopata A, Johnston IW, Hoult IJ, Speirs AI. Pregnancy following intrauterine implantation of an embryo obtained by in vitro fertilization of a preovulatory egg. Fertil Steril. 1980;33:117-20
31. Edwards RG, Steptoe PC, Purdy JM. Establishing full-term human pregnancies using cleaving embryos grown in vitro. Br J Obstet Gynaecol 1980;87:737-56
32. Ronaghi M, Karamohamed S, Pettersson B, Uhlen M, Nyren P. Real-time DNA sequencing using detection of pyrophosphate release. Anal Biochem 1996;242:84-9
33. Sasieni PD. From Genotypes to genes: doubling the sample size. Biometrics 1997;53:1253-61
34. Tregouet DA, Escolano S, Tiret L, Mallet A, Golmard JL. A new algorithm for haplotype-based association analysis: the Stochastic-EM algorithm. Ann Hum Genet 2004;68:165-77
35. Lewontin RC and Kojima K. The evolutionary dynamics of complex polymorphisms. Evolution 1960;14:450-72
36. Kozak M. Emerging links between initiation of translation and human diseases. Mamm Genome 2002;13:401-10
37. Kenis H, Doggen CJ, Vos HL, Reutelingsperger CP, vn Heerde WL. The C-1T mutation in the annexin A5 Kozak sequence slightly increases the risk of myocardial infarction in men. Thromb Haemost 2003;1:2688-9
38. Falany JL, Falany CN. Regulation of oestrogen activity by sulfation in human MCF-7 breast cancer cells. Oncol Res 1997;9:589-96
39. Ortmann O, Sturm R, Knuppen R, Emons G. Weak estrogenic activity of phenol red in the pituitary gonadotroph: re-evaluation of estrogen and antiestrogen effects. J Steroid Biochem 1990;35:17-22
40. Otsuka F and Shimasaki S. A negative feedback system between oocyte bone morphogenetic protein 15 and granulosa cell kit ligand: its role in regulating granulosa cell mitosis. Proc Natl Acad Sci USA 2002;99:8060-5
41. Ramenghi U, Ruggieri L, Dianzani I, Rosso C, Brizzi MF, Camaschella C, Pietsch T, Saglio G. Human peripheral blood granulocytes and myeloid leukemic cell lines express both transcripts encoding for stem cell factor. Stem Cells 1994;12:521-6
42. Bodin L, Lecerf F, Pisselet C, SanCristobal M, Bibe B, Mulsant P. How many mutations are associated with increased ovulation rate and litter size and progeny of Lacaune meat sheep? Workshop on Major Genes and QTL in Sheep and Goat, Toulouse, France, 8-11 December 2003
43. Hanrahan JP, Gregan SM, Mulsant P, Mullen M, Davis GH, Powell R, Galloway SM. Mutations in the genes for oocyte-derived growth factors GDF9 and BMP15 are associated with both increased ovulation rate and sterility in Cambridge and Belclare sheep (Ovis aries). Biol Reprod 2004;70:900-9
44. Yan C, Wang P, DeMayo J, DeMayo FJ, Elvin JA, Carino C, Prasad SV, Skinner SS, Dunbar BS, Dube JL, Celeste AJ, Matzuk MM. Synergistic roles of bone morphogenetic protein 15 and growth differentiation factor 9 in ovarian function. Mol Endocrinol 2001;15:854-66
45. Di Pasquale E, Beck-Peccoz P, Persani L. Hypergonadotropic ovarian failure associated with an inherited mutation of human bone morphogenetic protein-15 (BMP15) gene. Am J Hum Genet. 2004;75:106-11. Epub 2004 May 10
46. Otsuka F, Yao Z, Lee T, Yamamoto S, Erickson GF, Shimasaki S. Bone morphogenetic protein-15. Identification of target cells and biological functions. J Biol Chem 2000;275:39523-8
47. Otsuka F, Yamamoto S, Erickson GF, Shimasaki S. Bone morphogenetic protein-15 inhibits follicle-stimulating hormone (FSH) action by suppressing FSH receptor expression. J Biol Chem 2001;276:11387-92
48. Adashi EY, Resnick CE, Hurwitz A, Ricciarelli E, Hernandez ER, Roberts CT, Leroith D, Rosenfeld R. Insulin-like growth factors: the ovarian connection. Hum Reprod 1991;6:1213-9
49. Miro F, Hillier SG. Modulation of granulosa cell deoxyribonucleic acid synthesis and differentiation by activin. Endocrinology 1996;137:464-8
50. Thomas FH, Ethier JF, Shimasaki S, Vanderhyden BC. Follicle-stimulating hormone regulates oocyte growth by modulation of expression of oocyte and granulosa cell factors. Endocrinology 2005;146:941-9
51. Kruskal WH y Wallis HA. Use of ranks in one-criterion variance analisis". J. Amer Statist Assoc 1952;47:583-21; errata, ibid., 48:907-11
52. De Castro F, Moron F, Montoro L, Real LM, Ruiz A. Pharmacogenetics of controlled ovarian hyperstimulation. Pharmacogenet Genomics 2006;16:485-95
53. Lawson R, EI-Toukhy T, Kassab A, Taylor A, Braude P, Parsons J, Seed P. Poor response to ovulation induction is a stronger predictor of early menopause than elevated basal FSH: a life table analysis. Hum Reprod 2003;18:527-33
54. Nikolaou D, Lavery S, Turner C, Margara R, Trew G. Is there a link between an extremely poor response to ovarian hyperstimulation and early ovarian failure? Hum Reprod 2002;17:1106-11

### Description of the Figures

Figure 1A shows a diagram of *BMP15* gene with the polymorphisms studied. In figure 1B, we extend the 5'UTR region showing the 1 Kb sequence used in its functional studies. This region contains the four initial codons of gene and nucleotides up-stream from them. We show in black and underlined the *Xho*I and *Nco*I restriction sites used to extract this region, as well as the TATA box, ERE sequence and exact localization of nucleotides included in the polymorphic variants.
Figure 2 shows the plots obtained in the genotype analysis of -9C>G (upper figure) IVS1+905A>G (middle figure) and N103S (lower figure) polymorphisms.
Figure 3 shows the sequence chromatographs of the *BMP15* gene regions containing IVS1+905A>G (upper left image), N103S (upper right image), -9C>G (lower left image) and -673C>T (lower right image) polymorphisms.
Figure 4 shows a graphic representing estimated haplotype frequencies TGG-, CCA- and CCG- in patients with early OHSS (upper graphic) versus rest of patients (lower graphic).

## Claims

1. An *in vitro* method of detection of molecular markers within *BMP15* gene for the diagnosis or prognosis of a predisposition to develop an alteration in ovarian function in women selected among developing an ovarian hyperstimulation syndrome when a woman is underwent controlled ovarian stimulation, early menopause or premature ovarian failure, **characterized in that**, it detects the presence/absence of, at least, IVS1+905A>G polymorphism.

2. An *in vitro* method of detection of molecular markers within *BMP15* gene for the diagnosis or prognosis of a predisposition to develop an alteration in ovarian function in women according to claim 1, **characterized in that**, it detects additionally the presence/absence of, at least, a polymorphism selected from -673C>T, -9C>G and N103S.

3. An *in vitro* method of detection of molecular markers within *BMP15* gene for the diagnosis or prognosis of a predisposition to develop an alteration in ovarian function in women according to claims 1 or 2, **characterized in that**, detection of the presence of IVS1+905A>G polymorphism is considered indicative of predisposition to develop an alteration in ovarian function.

4. An *in vitro* method of detection of molecular markers within *BMP15* gene for the diagnosis or prognosis of a predisposition to develop an alteration in ovarian function in women according to claim 3, **characterized in that**, detection of the presence of IVS1+905A>G polymorphism in just one allele from the patient is considered indicative of predisposition to develop an alteration in ovarian function.

5. An *in vitro* method of detection of molecular markers within *BMP15* gene for the diagnosis or prognosis of a predisposition to develop an alteration in ovarian function in women according to claim 2, **characterized in that**, it detects the presence/absence of, at least, -673C>T, -9C>G and IVS1+905A>G polymorphisms.

6. An *in vitro* method of detection of molecular markers within *BMP15* gene for the diagnosis or prognosis of a predisposition to develop an alteration in ovarian function in women according to claim 5, **characterized in that**, it detects, at least, the consensus TGG- haplotype.

7. An *in vitro* method of detection of molecular markers within *BMP15* gene for the diagnosis or prognosis of a predisposition to develop an alteration in ovarian function in women according to claim 6, **characterized in that**, the detection of the presence of the consensus TGG- haplotype is considered indicative of predisposition to develop an alteration in ovarian function.

8. An *in vitro* method of detection of molecular markers within *BMP15* gene for the diagnosis or prognosis of a predisposition to develop an alteration in ovarian function in women according to claim 7, **characterized in that**, the detection of the presence of the consensus TGG- haplotype is considered indicative of predisposition to develop an alteration in ovarian function, which consists in developing an ovarian hyperstimulation syndrome when a woman is underwent controlled ovarian stimulation.

9. An *in vitro* method of detection of molecular markers within *BMP15* gene for the diagnosis or prognosis of a predisposition to develop an alteration in ovarian function in women according to claim 8, **characterized in that**, the detection of the presence of the consensus TGG- haplotype is considered indicative of predisposition to develop an alteration in ovarian function, which consists in developing early symptoms of the ovarian hyperstimulation syndrome when a woman is underwent controlled ovarian-stimulation.

10. An *in vitro* method of detection of molecular markers within *BMP15* gene for the diagnosis or prognosis of a predisposition to develop an alteration in ovarian function in women according to claim 5, **characterized in that**, it detects, at least, the consensus CCA- haplotype.

11. An *in vitro* method of detection of molecular markers within *BMP15* gene for the diagnosis or prognosis of a predisposition to develop an alteration in ovarian function in women according to claim 10, **characterized in that**, the detection of the presence of the consensus CCA- haplotype is considered a protective factor against predisposition to develop an alteration in ovarian function.

12. An *in vitro* method of detection of molecular markers within *BMP15* gene for the diagnosis or prognosis of a predisposition to develop an alteration in ovarian function in women according to claim 11, **characterized in that**, the detection of the presence of the consensus CCA- haplotype is considered a protective factor against predisposition to develop an alteration in ovarian function, which consists in developing an ovarian hyperstimulation syndrome when a woman is underwent controlled ovarian stimulation

13. An *in vitro* method of detection of molecular markers within *BMP15* gene for the diagnosis or prognosis of a predisposition to develop an alteration in ovarian function in women according to claim 12, **characterized in that**, the detection of the presence of the consensus CCA- haplotype is considered a protective factor against predisposition to develop an alteration in ovarian function, which consists in developing early symptoms of an ovarian hyperstimulation syndrome when a woman is underwent controlled ovarian stimulation

14. An *in vitro* method of detection of molecular markers within *BMP15* gene for the diagnosis or prognosis of a predisposition to develop of ari alteration in ovarian function in women according to claim 2, **characterized in that**, it detects, at least, the TGGA haplotype.

15. An *in vitro* method of detection of molecular markers within *BMP15* gene for the diagnosis or prognosis of a predisposition to develop an alteration in ovarian function in women according to claim 14, **characterized in that**, the detection of the presence of the TGGA haplotype is considered indicative of predisposition to develop an alteration in ovarian function.

16. An *in vitro* method of detection of molecular markers within *BMP15* gene for the diagnosis or prognosis of a predisposition to develop an alteration in ovarian function in women according to claim 15, **characterized in that**, the detection of the presence of the TGGA haplotype is considered indicative of predisposition to develop an alteration in ovarian function, which consists in developing an ovarian hyperstimulation syndrome when a woman is underwent controlled ovarian stimulation.

17. An *in vitro* method of detection of molecular markers within *BMP15* gene for the diagnosis or prognosis of a predisposition to develop an alteration in ovarian function in women according to claim 16, **characterized in that**, the detection of the presence of the TGGA haplotype is considered indicative of predisposition to develop an alteration in ovarian function, which consists in developing early symptoms of the ovarian hyperstimulation syndrome when a woman is underwent controlled ovarian stimulation.

18. An *in vitro* method of detection of molecular markers within *BMP15* gene for the diagnosis or prognosis of a predisposition to develop an alteration in ovarian function in women according to claim 2, **characterized in that**, it detects, at least, the CCAA haplotype.

19. An *in vitro* method of detection of molecular markers within *BMP15* gene for the diagnosis or prognosis of a predisposition to develop an alteration in ovarian function in women according to claim 18, **characterized in that**, the detection of the presence of the CCAA haplotype is considered a protective factor against predisposition to develop an alteration in ovarian function.

20. An *in vitro* method of detection of molecular markers within *BMP15* gene for the diagnosis or prognosis of a predisposition to develop an alteration in ovarian function in women according to claim 19, **characterized in that**, the detection of the presence of the CCAA haplotype is considered a protective factor against predisposition to develop an alteration in ovarian function, which consists in developing an ovarian hyperstimulation syndrome when a woman is underwent controlled ovarian stimulation.

21. An *in vitro* method of detection of molecular markers within *BMP15* gene for the diagnosis or prognosis of a predisposition to develop an alteration in ovarian function in women according to claim 20, **characterized in that**, the detection of the presence of the CCAA haplotype is considered a protective factor against predisposition to develop an alteration -in ovarian function, which consists in developing early symptoms of an ovarian hyperstimulation syndrome when a woman is underwent controlled ovarian stimulation.

## Patentansprüche

1. *In-vitro*-Verfahren zum Nachweis molekularer Marker innerhalb des *BMP15*-Gens zur Diagnose oder Prognose einer Veranlagung zur Entwicklung einer Funktionsstörung der Eierstöcke bei Frauen, ausgewählt aus Entwicklung eines ovariellen Überstimulationssyndroms bei einer Frau, die einer kontrollierten ovariellen Stimulation unterzogen wird, frühe Menopause oder vorzeitige Ovarialinsuffizienz, **dadurch gekennzeichnet, dass** es die An-/Abwesenheit mindestens eines IVS1+905A>G-Polymorphismus nachweist.

2. *In-vitro-*Verfahren zum Nachweis molekularer Marker innerhalb des *BMP15*-Gens zur Diagnose oder Prognose einer Veranlagung zur Entwicklung einer Funktionsstörung der Eierstöcke bei Frauen gemäß Anspruch 1, **dadurch gekennzeichnet, dass** es zusätzlich die An-/Abwesenheit mindestens eines Polymorphismus, ausgewählt aus -673C>T, -9C>G und N103S, nachweist.

3. *In-vitro*-Verfahren zum Nachweis molekularer Marker innerhalb des *BMP15*-Gens zur Diagnose oder Prognose einer Veranlagung zur Entwicklung einer Funktionsstörung der Eierstöcke bei Frauen gemäß Anspruch 1 oder 2, **dadurch gekennzeichnet, dass** der Nachweis der Anwesenheit eines IVS1+905A>G-Polymorphismus als Hinweis auf eine Veranlagung zur Entwicklung einer Funktionsstörung der Eierstöcke gilt.

4. *In-vitro*-Verfahren zum Nachweis molekularer Marker innerhalb des *BMP15-*Gens zur Diagnose oder Prognose einer Veranlagung zur Entwicklung einer Funktionsstörung der Eierstöcke bei Frauen gemäß Anspruch 3, **dadurch gekennzeichnet, dass** der Nachweis der Anwesenheit eines IVS1+905A>G-Polymorphismus in nur einem Allel der Patientin als Hinweis auf eine Veranlagung zur Entwicklung einer Funktionsstörung der Eierstöcke gilt.

5. *In-vitro*-Verfahren zum Nachweis molekularer Marker innerhalb des *BMP15*-Gens zur Diagnose oder Prognose einer Veranlagung zur Entwicklung einer Funktionsstörung der Eierstöcke bei Frauen gemäß Anspruch 2, **dadurch gekennzeichnet, dass** es die An-/Abwesenheit mindestens eines -673C>T-, - 9C>G- und IVS1+905A>G-Polymorphismus nachweist.

6. *In-vitro*-Verfahren zum Nachweis molekularer Marker innerhalb des *BMP15*-Gens zur Diagnose oder Prognose einer Veranlagung zur Entwicklung einer Funktionsstörung der Eierstöcke bei Frauen gemäß Anspruch 5, **dadurch gekennzeichnet, dass** es mindestens den Konsensus TGG-Haplotyp nachweist.

7. *In-vitro*-Verfahren zum Nachweis molekularer Marker innerhalb des *BMP15*-Gens zur Diagnose oder Prognose einer Veranlagung zur Entwicklung einer Funktionsstörung der Eierstöcke bei Frauen gemäß Anspruch 6, **dadurch gekennzeichnet, dass** der Nachweis der Anwesenheit des Konsensus TGG-Haplotyps als Hinweis auf eine Veranlagung zur Entwicklung einer Funktionsstörung der Eierstöcke gilt.

8. *In-vitro-*Verfahren zum Nachweis molekularer Marker innerhalb des *BMP15*-Gens zur Diagnose oder Prognose einer Veranlagung zur Entwicklung einer Funktionsstörung der Eierstöcke bei Frauen gemäß Anspruch 7, **dadurch gekennzeichnet, dass** der Nachweis der Anwesenheit des Konsensus TGG-Haplotyps als Hinweis auf eine Veranlagung zur Entwicklung einer Funktionsstörung der Eierstöcke gilt, die in der Entwicklung eines ovariellen Überstimulationssyndroms bei einer Frau, die einer kontrollierten ovariellen Stimulation unterzogen wird, besteht.

9. *In-vitro*-Verfahren zum Nachweis molekularer Marker innerhalb des *BMP15*-Gens zur Diagnose oder Prognose einer Veranlagung zur Entwicklung einer Funktionsstörung der Eierstöcke bei Frauen gemäß Anspruch 8, **dadurch gekennzeichnet, dass** der Nachweis der Anwesenheit des Konsensus TGG-Haplotyps als Hinweis auf eine Veranlagung zur Entwicklung einer Funktionsstörung der Eierstöcke gilt, die in der Entwicklung früher Symptome des ovariellen Überstimulationssyndroms bei einer Frau, die einer kontrollierten ovariellen Stimulation unterzogen wird, besteht.

10. *In-vitro*-Verfahren zum Nachweis molekularer Marker innerhalb des *BMP15*-Gens zur Diagnose oder Prognose einer Veranlagung zur Entwicklung einer Funktionsstörung der Eierstöcke bei Frauen gemäß Anspruch 5, **dadurch gekennzeichnet, dass** es mindestens den Konsensus CCA-Haplotyp nachweist.

11. *In-vitro*-Verfahren zum Nachweis molekularer Marker innerhalb des *BMP15*-Gens zur Diagnose oder Prognose einer Veranlagung zur Entwicklung einer Funktionsstörung der Eierstöcke bei Frauen gemäß Anspruch 10, **dadurch gekennzeichnet, dass** der Nachweis der Anwesenheit des Konsensus CCA-Haplotyps als Schutzfaktor gegen eine Veranlagung zur Entwicklung einer Funktionsstörung der Eierstöcke gilt.

12. *In-vitro*-Verfahren zum Nachweis molekularer Marker innerhalb des *BMP15*-Gens zur Diagnose oder Prognose einer Veranlagung zur Entwicklung einer Funktionsstörung der Eierstöcke bei Frauen gemäß Anspruch 11, **dadurch gekennzeichnet, dass** der Nachweis der Anwesenheit des Konsensus CCA-Haplotyps als Schutzfaktor gegen eine Veranlagung zur Entwicklung einer Funktionsstörung der Eierstöcke gilt, die in der Entwicklung eines ovariellen Überstimulationssyndroms bei einer Frau, die einer kontrollierten ovariellen Stimulation unterzogen wird, besteht.

13. *In-vitro*-Verfahren zum Nachweis molekularer Marker innerhalb des *BMP15*-Gens zur Diagnose oder Prognose einer Veranlagung zur Entwicklung einer Funktionsstörung der Eierstöcke bei Frauen gemäß Anspruch 12, **dadurch gekennzeichnet, dass** der Nachweis der Anwesenheit des Konsensus CCA-Haplotyps als Schutzfaktor gegen eine Veranlagung zur Entwicklung einer Funktionsstörung der Eierstöcke gilt, die in der Entwicklung früher Symptome eines ovariellen Überstimulationssyndroms bei einer Frau, die einer kontrollierten ovariellen Stimulation unterzogen wird, besteht.

14. *In-vitro*-Verfahren zum Nachweis molekularer Marker innerhalb des *BMP15*-Gens zur Diagnose oder Prognose einer Veranlagung zur Entwicklung einer Funktionsstörung der Eierstöcke bei Frauen gemäß Anspruch 2, **dadurch gekennzeichnet, dass** es mindestens den TGGA-Haplotyp nachweist.

15. *In-vitro*-Verfahren zum Nachweis molekularer Marker innerhalb des *BMP15*-Gens zur Diagnose oder Prognose einer Veranlagung zur Entwicklung einer Funktionsstörung der Eierstöcke bei Frauen gemäß Anspruch 14, **dadurch gekennzeichnet, dass** der Nachweis der Anwesenheit des TGGA-Haplotyps als Hinweis auf eine Veranlagung zur Entwicklung einer Funktionsstörung der Eierstöcke gilt.

16. *In-vitro*-Verfahren zum Nachweis molekularer Marker innerhalb des *BMP15*-Gens zur Diagnose oder Prognose einer Veranlagung zur Entwicklung einer Funktionsstörung der Eierstöcke bei Frauen gemäß Anspruch 15, **dadurch gekennzeichnet, dass** der Nachweis der Anwesenheit des TGGA-Haplotyps als Hinweis auf eine Veranlagung zur Entwicklung einer Funktionsstörung der Eierstöcke gilt, die in der Entwicklung eines ovariellen Überstimulationssyndroms bei einer Frau, die einer kontrollierten ovariellen Stimulation unterzogen wird, besteht.

17. *In-vitro*-Verfahren zum Nachweis molekularer Marker innerhalb des *BMP15*-Gens zur Diagnose oder Prognose einer Veranlagung zur Entwicklung einer Funktionsstörung der Eierstöcke bei Frauen gemäß Anspruch 16, **dadurch gekennzeichnet, dass** der Nachweis der Anwesenheit des TGGA-Haplotyps als Hinweis auf eine Veranlagung zur Entwicklung einer Funktionsstörung der Eierstöcke gilt, die in der Entwicklung früher Symptome eines ovariellen Überstimulationssyndroms bei einer Frau, die einer kontrollierten ovariellen Stimulation unterzogen wird, besteht.

18. *In-vitro*-Verfahren zum Nachweis molekularer Marker innerhalb des *BMP15*-Gens zur Diagnose oder Prognose einer Veranlagung zur Entwicklung einer Funktionsstörung der Eierstöcke bei Frauen gemäß Anspruch 2, **dadurch gekennzeichnet, dass** es mindestens den CCAA-Haplotyp nachweist.

19. *In-vitro*-Verfahren zum Nachweis molekularer Marker innerhalb des *BMP15*-Gens zur Diagnose oder Prognose einer Veranlagung zur Entwicklung einer Funktionsstörung der Eierstöcke bei Frauen gemäß Anspruch 18, **dadurch gekennzeichnet, dass** der Nachweis der Anwesenheit des CCAA-Haplotyps als Schutzfaktor gegen eine Veranlagung zur Entwicklung einer Funktionsstörung der Eierstöcke gilt.

20. *In-vitro*-Verfahren zum Nachweis molekularer Marker innerhalb des *BMP15*-Gens zur Diagnose oder Prognose einer Veranlagung zur Entwicklung einer Funktionsstörung der Eierstöcke bei Frauen gemäß Anspruch 19, **dadurch gekennzeichnet, dass** der Nachweis der Anwesenheit des CCAA-Haplotyps als Schutzfaktor gegen eine Veranlagung zur Entwicklung einer Funktionsstörung der Eierstöcke gilt, die in der Entwicklung eines ovariellen Überstimulationssyndrom bei einer Frau, die einer kontrollierten ovariellen Stimulation unterzogen wird, besteht.

21. *In-vitro*-Verfahren zum Nachweis molekularer Marker innerhalb des *BMP15*-Gens zur Diagnose oder Prognose einer Veranlagung zur Entwicklung einer Funktionsstörung der Eierstöcke bei Frauen gemäß Anspruch 20, **dadurch gekennzeichnet, dass** der Nachweis der Anwesenheit des CCAA-Haplotyps als Schutzfaktor gegen eine Veranlagung zur Entwicklung einer Funktionsstörung der Eierstöcke gilt, die in der Entwicklung früher Symptome eines ovariellen Überstimulationssyndrom bei einer Frau, die einer kontrollierten ovariellen Stimulation unterzogen wird, besteht.

## Revendications

1. Un procédé in vitro de détection de marqueurs moléculaires dans le gène BMP15 pour le diagnostic ou le pronostic d'une prédisposition à développer une altération de la fonction ovarienne chez des femmes sélectionnées parmi celles développant un syndrome d'hyperstimulation ovarienne lorsqu'une femme a subi une stimulation ovarienne contrôlée, une ménopause précoce ou une insuffisance ovarienne prématurée, **caractérisé en ce qu'**il détecte la présence/absence, d'au moins, le polymorphisme IVS1+905A>G.

2. Un procédé in vitro de détection de marqueurs moléculaires dans le gène BMP15 pour le diagnostic ou le pronostic d'une prédisposition à développer une altération de la fonction ovarienne chez des femmes selon la revendication 1, **caractérisé en ce qu'**il détecte en outre la présence/absence, d'au moins, un polymorphisme sélectionné parmi -673C>T, -9C>G et N103S.

3. Un procédé in vitro de détection de marqueurs moléculaires dans le gène BMP15 pour le diagnostic ou le pronostic d'une prédisposition à développer une altération de la fonction ovarienne chez des femmes selon les revendications 1 ou 2, **caractérisé en ce que**, la détection de la présence du polymorphisme IVS1+905A>G est considérée comme indicative de la prédisposition à développer une altération de la fonction ovarienne.

4. Un procédé in vitro de détection de marqueurs moléculaires dans le gène BMP15 pour le diagnostic ou le pronostic d'une prédisposition à développer une altération de la fonction ovarienne chez des femmes selon la revendication 3, **caractérisé en ce que**, la détection de la présence du polymorphisme IVS1+905A>G dans un seul allèle du patient est considérée comme indicative de la prédisposition à développer une altération de la fonction ovarienne.

5. Un procédé in vitro de détection de marqueurs moléculaires dans le gène BMP15 pour le diagnostic ou le pronostic d'une prédisposition à développer une altération de la fonction ovarienne chez des femmes selon la revendication 2, **caractérisé en ce qu'**il détecte la présence/absence, d'au moins, les polymorphismes -673C>T, -9C>G et IVS1+905A>G.

6. Un procédé in vitro de détection de marqueurs moléculaires dans le gène BMP15 pour le diagnostic ou le pronostic d'une prédisposition à développer une altération de la fonction ovarienne chez des femmes selon la revendication 5, **caractérisé en ce qu'**il détecte, au moins, l'haplotype consensus TGG-.

7. Un procédé in vitro de détection de marqueurs moléculaires dans le gène BMP15 pour le diagnostic ou le pronostic d'une prédisposition à développer une altération de la fonction ovarienne chez des femmes selon la revendication 6, **caractérisé en ce que**, la détection de la présence de l'haplotype consensus TGG- est considérée comme indicative de la prédisposition à développer une altération de la fonction ovarienne.

8. Un procédé in vitro de détection de marqueurs moléculaires dans le gène BMP15 pour le diagnostic ou le pronostic d'une prédisposition à développer une altération de la fonction ovarienne chez des femmes selon la revendication 7, **caractérisé en ce que**, la détection de la présence de l'haplotype consensus TGG- est considérée comme indicative de la prédisposition à développer une altération de la fonction ovarienne, qui consiste à développer un syndrome d'hyperstimulation ovarienne lorsqu'une femme a subi une stimulation ovarienne contrôlée.

9. Un procédé in vitro de détection de marqueurs moléculaires dans le gène BMP15 pour le diagnostic ou le pronostic d'une prédisposition à développer une altération de la fonction ovarienne chez des femmes selon la revendication 8, **caractérisé en ce que**, la détection de la présence de l'haplotype consensus TGG- est considérée comme indicative de la prédisposition à développer une altération de la fonction ovarienne, qui consiste à développer des symptômes précoces du syndrome d'hyperstimulation ovarienne lorsqu'une femme a subi une stimulation ovarienne contrôlée.

10. Un procédé in vitro de détection de marqueurs moléculaires dans le gène BMP15 pour le diagnostic ou le pronostic d'une prédisposition à développer une altération de la fonction ovarienne chez des femmes selon la revendication 5, **caractérisé en ce qu'**il détecte, au moins, l'haplotype consensus CCA-.

11. Un procédé in vitro de détection de marqueurs moléculaires dans le gène BMP15 pour le diagnostic ou le pronostic d'une prédisposition à développer une altération de la fonction ovarienne chez des femmes selon la revendication 10, **caractérisé en ce que**, la détection de la présence de l'haplotype consensus CCA- est considérée comme un facteur protecteur contre la prédisposition à développer une altération de la fonction ovarienne.

12. Un procédé in vitro de détection de marqueurs moléculaires dans le gène BMP15 pour le diagnostic ou le pronostic d'une prédisposition à développer une altération de la fonction ovarienne chez des femmes selon la revendication 11, **caractérisé en ce que**, la détection de la présence de l'haplotype consensus CCA- est considérée comme un facteur protecteur contre la prédisposition à développer une altération de la fonction ovarienne, qui consiste à développer un syndrome d'hyperstimulation ovarienne lorsqu'une femme a subi une stimulation ovarienne contrôlée.

13. Un procédé in vitro de détection de marqueurs moléculaires dans le gène BMP15 pour le diagnostic ou le pronostic d'une prédisposition à développer une altération de la fonction ovarienne chez des femmes selon la revendication 12, **caractérisé en ce que**, la détection de la présence de l'haplotype consensus CCA- est considérée comme un facteur protecteur contre la prédisposition à développer une altération de la fonction ovarienne, qui consiste à développer des symptômes précoces d'un syndrome d'hyperstimulation ovarienne lorsqu'une femme a subi une stimulation ovarienne contrôlée.

14. Un procédé in vitro de détection de marqueurs moléculaires dans le gène BMP15 pour le diagnostic ou le pronostic d'une prédisposition à développer une altération de la fonction ovarienne chez des femmes selon la revendication 2, **caractérisé en ce qu'**il détecte, au moins, l'haplotype TGGA.

15. Un procédé in vitro de détection de marqueurs moléculaires dans le gène BMP15 pour le diagnostic ou le pronostic d'une prédisposition à développer une altération de la fonction ovarienne chez des femmes selon la revendication 14, **caractérisé en ce que**, la détection de la présence de l'haplotype TGGA est considérée comme indicative de la prédisposition à développer une altération de la fonction ovarienne.

16. Un procédé in vitro de détection de marqueurs moléculaires dans le gène BMP15 pour le diagnostic ou le pronostic d'une prédisposition à développer une altération de la fonction ovarienne chez des femmes selon la revendication 15, **caractérisé en ce que**, la détection de la présence de l'haplotype TGGA est considérée comme indicative de la prédisposition à développer une altération de la fonction ovarienne, qui consiste à développer un syndrome d'hyperstimulation ovarienne lorsqu'une femme a subi une stimulation ovarienne contrôlée.

17. Un procédé in vitro de détection de marqueurs moléculaires dans le gène BMP15 pour le diagnostic ou le pronostic d'une prédisposition à développer une altération de la fonction ovarienne chez des femmes selon la revendication 16, **caractérisé en ce que**, la détection de la présence de l'haplotype TGGA est considérée comme indicative de la prédisposition à développer une altération de la fonction ovarienne, qui consiste à développer des symptômes précoces du syndrome d'hyperstimulation ovarienne lorsqu'une femme a subi une stimulation ovarienne contrôlée.

18. Un procédé in vitro de détection de marqueurs moléculaires dans le gène BMP15 pour le diagnostic ou le pronostic d'une prédisposition à développer une altération de la fonction ovarienne chez des femmes selon la revendication 2, **caractérisé en ce qu'**il détecte, au moins, l'haplotype CCAA.

19. Un procédé in vitro de détection de marqueurs moléculaires dans le gène BMP15 pour le diagnostic ou le pronostic d'une prédisposition à développer une altération de la fonction ovarienne chez des femmes selon la revendication 18, **caractérisé en ce que**, la détection de la présence de l'haplotype CCAA est considérée comme un facteur protecteur contre la prédisposition à développer une altération de la fonction ovarienne.

20. Un procédé in vitro de détection de marqueurs moléculaires dans le gène BMP15 pour le diagnostic ou le pronostic d'une prédisposition à développer une altération de la fonction ovarienne chez des femmes selon la revendication 19, **caractérisé en ce que**, la détection de la présence de l'haplotype CCAA est considérée comme un facteur protecteur contre la prédisposition à développer une altération de la fonction ovarienne, qui consiste à développer un syndrome d'hyperstimulation ovarienne lorsqu'une femme a subi une stimulation ovarienne contrôlée.

21. Un procédé in vitro de détection de marqueurs moléculaires dans le gène BMP15 pour le diagnostic ou le pronostic d'une prédisposition à développer une altération de la fonction ovarienne chez des femmes selon la revendication 20, **caractérisé en ce que**, la détection de la présence de l'haplotype CCAA est considérée comme un facteur protecteur contre la prédisposition à développer une altération de la fonction ovarienne, qui consiste à développer des symptômes précoces d'un syndrome d'hyperstimulation ovarienne lorsqu'une femme a subi une stimulation ovarienne contrôlée.
